# EUROPEAN PATENT APPLICATION

(11) **EP 4 400 114 A1**
(43) Date of publication of application: **17.07.2024**
(21) Application number: 22864771.5
(22) Date of filing: 06.09.2022
(51) Int. Cl.: A61K 39/00, A61K 45/00, A61K 45/06, A61P 35/00, A61P 37/04, C07K 7/06, C12N 15/11, A61K 38/03, A61K 31/427, A61K 31/52

(54) **IMPROVEMENT OF TUMOR IMMUNOGENICITY BY MEANS OF SPLICING-CONTROLLING COMPOUND**

(30) Priority: 06.09.2021 JP 2021144961
(71) Applicant: Kyoto University, Kyoto-shi, Kyoto 606-8501 (JP)
(72) Inventor: HAGIWARA Masatoshi, Kyoto 606-8501 (JP); AJIRO Masahiko, Kyoto 606-8501 (JP); MATSUSHIMA Shingo, Kyoto 606-8501 (JP)
(74) Representative: Dehns
(86) International application number: PCT/JP2022/033464
(87) International publication number: WO 2023/033189

(57) **Abstract**

Provided is a pharmaceutical composition that can improve tumor immunogenicity. The present disclosure relates to a pharmaceutical composition containing, as an active ingredient, a splicing-controlling compound that modulates the activity of serine/arginine-rich splicing factors (SRSFs).

## Description

### Technical Field

The present disclosure relates to improvement of tumor immunogenicity and enhancement of immune checkpoint therapy by means of a splicing-controlling compound, as well as a pharmaceutical composition and a method therefor.

### Background Art

Neoantigens play an important role in CD8⁺ T cell-dependent immune surveillance and subsequent tumor killing Immune checkpoint therapy, which strengthens the host's immune system, has demonstrated impressive therapeutic efficacy against various malignancies. However, a non-negligible proportion of patients are poorly responsive or are resistant to immune checkpoint therapy, and new approaches are needed to enhance the efficacy of immune checkpoint therapy. Response to immune checkpoint therapy is associated with the immunogenicity of the tumor, which is determined primarily by the amount of neoantigens. Indeed, recent clinical studies have shown that tumor mutational burden (TMB), which is an indirect measure of neoantigen load, correlates with responsiveness to PD-1 blockage therapy. Furthermore, tumors of patients with primary or acquired resistance to immune checkpoint therapy often contain mutations in MHC class I-related genes such as JAK1/2 and B2M, which are responsible for defective presentation of neoantigens. These findings suggest that tumor immunogenicity with appropriate neoantigen loading is quite important for response to immune checkpoint therapy.

With the development of next-generation sequencer technology in recent years, new types of neoantigens that go beyond conventional concepts have been proposed. Comprehensive analysis using the Cancer Genome Atlas (TCGA) database revealed that abnormal pre-mRNA splicing events occur frequently in various cancers. Among them, there may be neoepitopes that translate potential neoantigens. Therefore, attempts are being made to test whether these "splice-neoantigens" are actually produced in cancer cells and contribute to immunogenicity. As a proof of this concept, Bigot et al. reported that CD8⁺ T cells recognizing immunogenic splice-neoantigens were present in peripheral blood and tumors of uveal melanoma patients with mutations of the splicing factor SF3B1, and that these CD8⁺ T cell clones specifically recognized and killed SF3B1-mutated tumor cells (Non-Patent Document 1). These results indicate that altering the RNA splicing pattern in tumor cells to produce splice-neoantigens is key to managing tumor immunogenicity and support that this splicing alteration is one therapeutic target in cancer immunotherapy.

Recently, a paper entitled "Pharmacologic modulation of RNA splicing enhances anti-tumor immunity" was published (Non-Patent Document 2). This document showed that use of RNA splicing modulators Indisulam, which act on the splicing factor RBM39, and MS-023 diversely alters splicing events and generates splice-neoantigens in tumor cells.

### Prior Art Documents

### Non-Patent Documents

[Non-Patent Document 1] Cancer Discov. 2021 Apr 2. doi: 10.1158/2159-8290.CD-20-0555.
[Non-Patent Document 2] Cell. 2021 Jul 22;184(15)4032-4047.e31.

### Disclosure of Invention

### Problem to be Solved by the Invention

The splicing patterns affected by RNA splicing modulators depend on the molecular target. An RNA splicing modulator whose molecular target is a splicing factor different from the molecular target of the RNA splicing modulators disclosed in Non-Patent Document 2 could improve tumor immunogenicity due to its own repertoire of splice-neoantigens. Use of a combination of a plurality of RNA splicing modulators whose molecular targets are different splicing factors is expected to synergistically enhance the tumor immunogenicity.

### Means for Solving Problem

In one aspect, the present disclosure relates to a pharmaceutical composition for use in improving tumor immunogenicity, the pharmaceutical composition containing, as an active ingredient, a splicing-controlling compound that modulates the activity of serine/arginine-rich splicing factors (SRSFs).

In one aspect, the present disclosure relates to a method for improving tumor immunogenicity, the method including bringing a splicing-controlling compound that modulates the activity of serine/arginine-rich splicing factors (SRSFs) into contact with a tumor.

In one aspect, the present disclosure relates to a method for improving tumor immunogenicity, the method including bringing a pharmaceutical composition according to the present disclosure into contact with a tumor.

In one aspect, the present disclosure relates to a method for enhancing immune checkpoint therapy, the method including administering simultaneously, separately, or sequentially an immune checkpoint inhibitor and a pharmaceutical composition according to the present disclosure in combination.

In one aspect, the present disclosure relates to a kit for use in cancer immunotherapy, the kit including a pharmaceutical composition according to the present and an immune checkpoint inhibitor.

In one aspect, the present disclosure relates to use of a combination of a pharmaceutical composition according to the present disclosure and an immune checkpoint inhibitor, for cancer immunotherapy.

In one aspect, the present disclosure relates to use of a splicing-controlling compound that modulates the activity of serine/arginine-rich splicing factors (SRSFs), for production of a pharmaceutical composition for improving tumor immunogenicity.

In one aspect, the present disclosure relates to a method for producing a splice-neoantigen, the method including bringing a tumor and a splicing-controlling compound of the present disclosure into contact with each other.

In one aspect, the present disclosure relates to a pharmaceutical composition for use in improving immunogenicity of a tumor, the pharmaceutical composition containing, as an active ingredient, a splice-neoantigen produced by the tumor in contact with a splicing-controlling compound of the present disclosure.

### Effects of the Invention

According to the present disclosure, in one or more embodiments, tumor immunogenicity can be improved.

According to the present disclosure, in one or more embodiments, a new splice-neoantigen can be induced in a tumor.

According to the present disclosure, in one or more embodiments, immune checkpoint therapy can be enhanced.

### Brief Description of Drawings

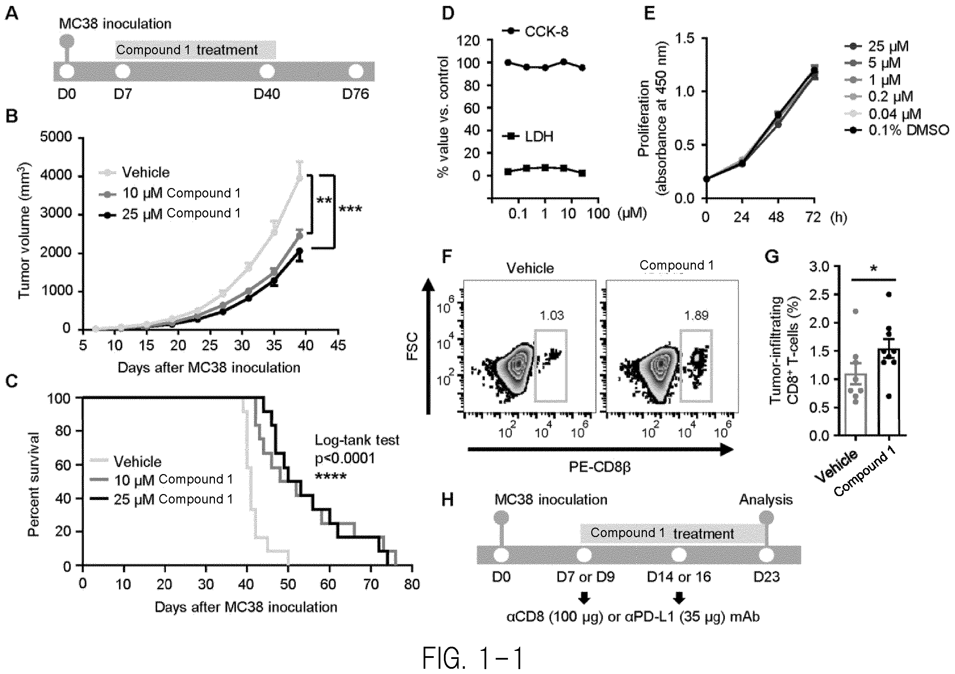

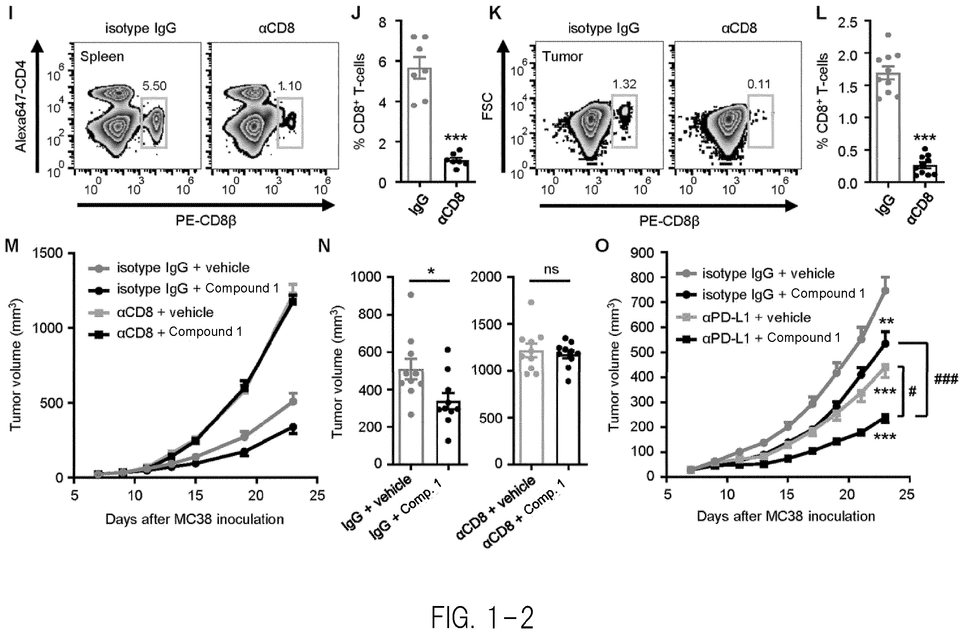

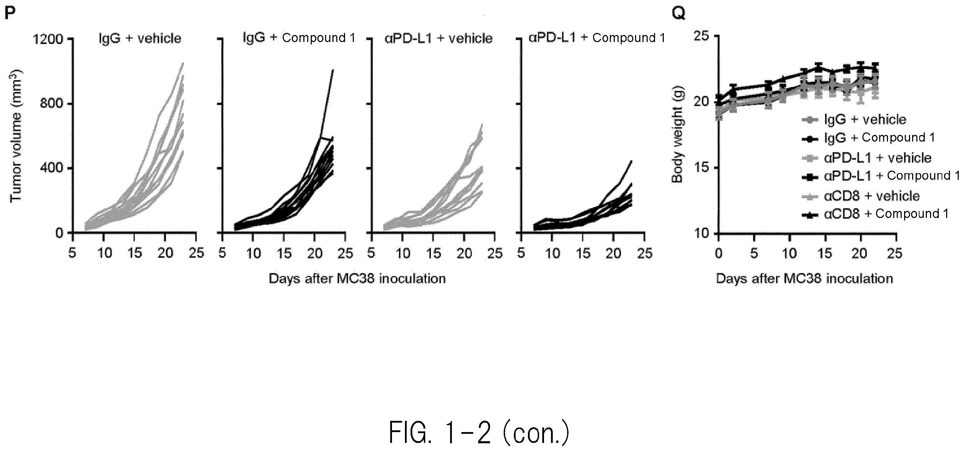

[FIG. 1-1] FIG. 1. Chemical activation of SRSFs exerts antitumor effects in a CD8⁺ T cell-dependent manner and potentiates the response to PD-1 blockade. A: Experimental procedure involving an MC38 tumor-bearing model. B and C: Tumor volumes (B) and survival (C) are shown (n = 12/group). ** represents p < 0.01; and *** represents p < 0.001. D and E: MC38 cells were treated with Compound 1 for 72 hours (D) or for the indicated time (E). Cell proliferation and cell death were measured using CCK-8 and LDH assays, respectively (n = 3/group). F and G: Representative images (F) and quantitative data (G) of flow cytometry are shown (n = 89/group). * represents p < 0.05. H: Experimental procedure of antibody injection in an MC38 tumor-bearing model.
[FIG. 1-2] Continuation of FIG. 1. I to L: Representative images (I and K) and quantitative data (J and L) of flow cytometry are shown (n = 7/group for spleen (J); n = 10/group for tumor (L)). *** represents p < 0.001. M and N: Tumor growth curves (M) and tumor volumes on day 23 (N) are shown (n = 10/group). * represents p < 0.05. "ns" represents "not significant". O and P: Tumor volumes of mice (n = 12/group) are shown collectively (O) or individually (P). ** represents p < 0.01, and *** represents p < 0.001, vs. isotype IgG + vehicle. # represents p < 0.05, and ### represents p < 0.001, between the indicated groups. Q: Changes in body weight of mice (n = 5/group). In FIG. 1, all data are shown as the mean ± SEM. All experiments were performed at least twice. Statistical significance was determined using unpaired Student's t-test for (G), (J), (L), and (N), and one-way ANOVA followed by Bonferroni's multiple comparisons test for (B) and (O).

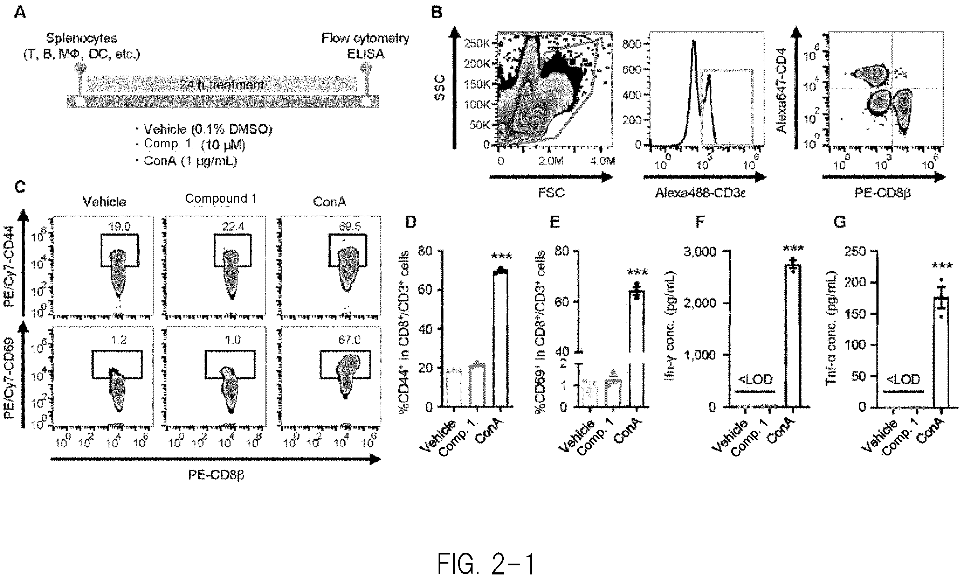

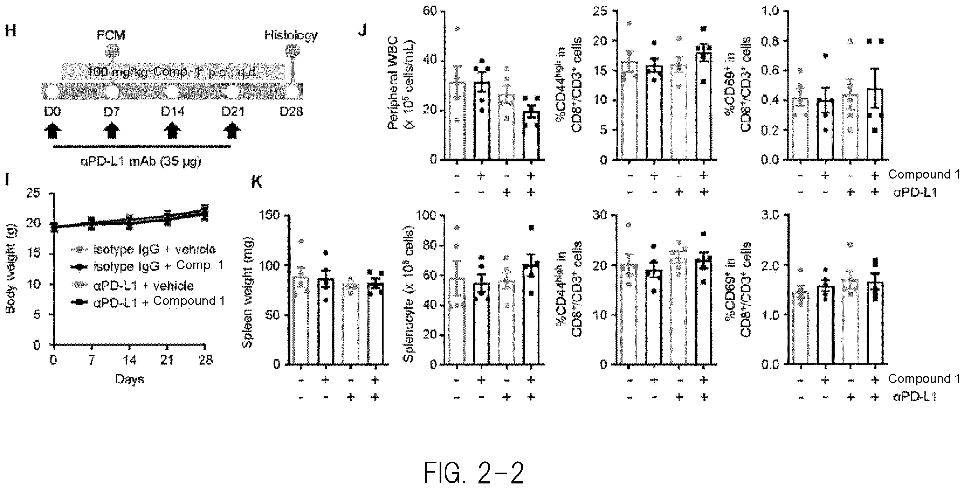

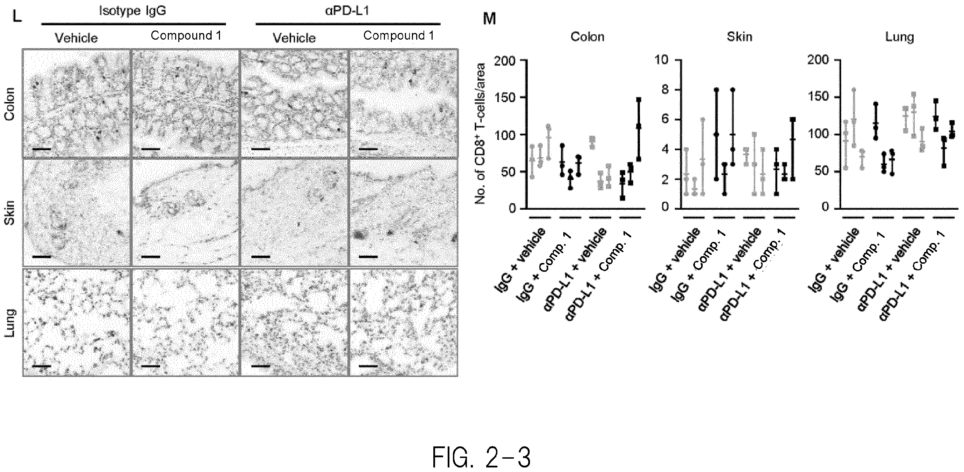

[FIG. 2-1] FIG. 2. Compound 1 enhances tumor immunogenicity without antigen-independent activation of T cells or autoimmune reactions. A: Experimental procedure of an ex vivo splenocyte assay. Splenocytes were prepared from 8-week-old female C57BL/6N mice and treated with Compound 1 for 24 hours. B to E: Frequencies of CD44⁺ and CD69⁺ among CD8⁺ T cells were measured through flow cytometry, and representative images (B and C) and quantitative data (D and E) are shown (n = 3/group). F and G: The concentrations of IFN-y (F) and TNF-α (G) in supernatant were measured (n = 3/group). "LOD" represents the "limit of detection".
[FIG. 2-2] Continuation of FIG. 2. H to M: Compound 1 (100 mg/kg) or vehicle control (0.5% CMC-Na) was orally administered to 7-week-old female C57BL/6N mice twice daily for 7 continuous days (J and K) or once daily for 4 weeks (I, L, and M), while αPD-L1 antibody or isotype control (isotype IgG) was intraperitoneally injected once weekly. The experimental scheme is indicated in (H). Body weights (n = 6/group) were monitored weekly (I). Mice were sacrificed on day 7 (n = 5/group) to analyze peripheral blood (J) and spleen (K).
[FIG. 2-3] Continuation of FIG. 2. L and M: Mice were sacrificed on day 28 (n = 3/group) to perform CD8 immunohistochemical staining (IHC). Scale bars represent 50 µm. The number of CD8⁺ cells was counted using Imaged software in three areas of each tissue. All data are shown as the mean ± SEM. Experiments were performed at least twice for B to G, or once for others. *** p < 0.001 vs. vehicle control was determined using one-way ANOVA followed by Bonferroni's multiple comparisons test.

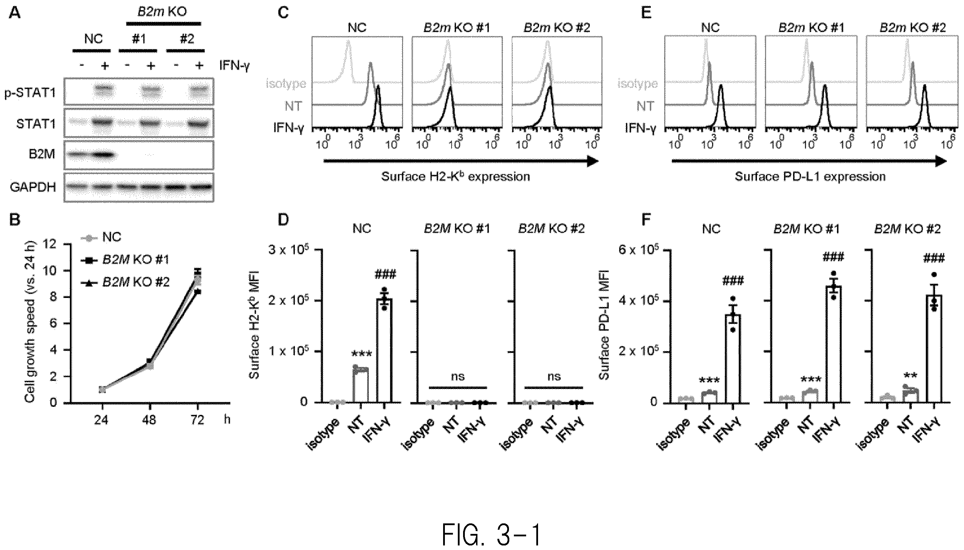

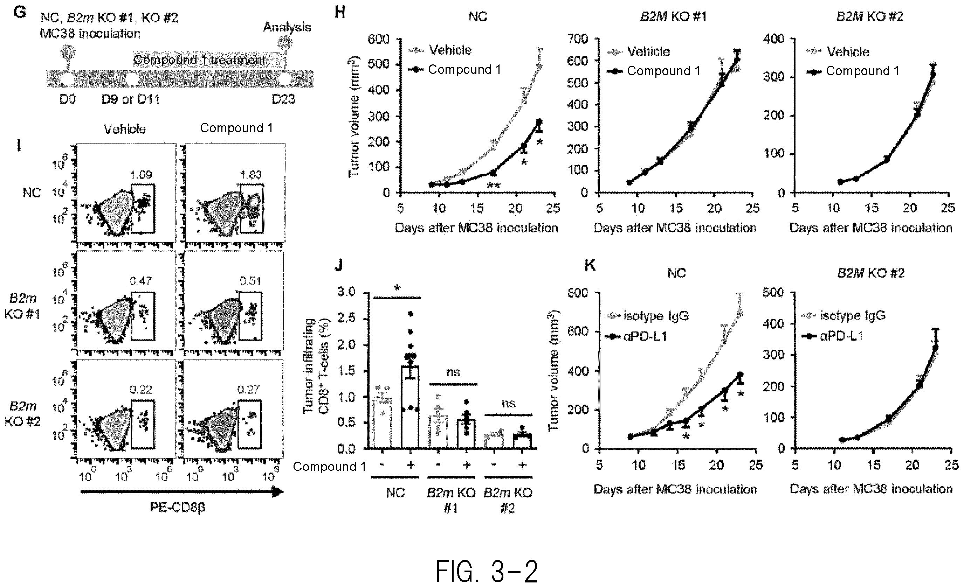

[FIG. 3-1] FIG. 3. MHC class I in tumor cells is indispensable for the antitumor effects of Compound 1. A: Western blotting analysis of indicated proteins in cell lysates from non-targeting control (NC) and B2m knockout clones (B2m KO #1 and #2) derived from MC38 cells treated with 10 ng/mL IFN-γ for 24 hours. GAPDH served as a loading control. B: Cell proliferation was measured using CCK-8 assay (n = 3/group). C to F: Each clone was treated with 1 ng/mL IFN-y for 24 hours, and the expression of H2-Kb (flow cytometric images (C) and quantification (D)) and PD-L1 (flow cytometric images (E) and quantification (F)) on the cell surface were analyzed (n = 3/group). ** represents p < 0.01, and *** represents p < 0.001, vs. isotype IgG control. ### represents p < 0.001 vs. nontreatment (NT).
[FIG. 3-2] Continuation of FIG. 3. G to J: The experimental schema (G), tumor volumes (H), flow cytometric images (I), as well as quantitative data (J) of tumor-infiltrating CD8⁺ T cells on day 23 are shown (n = 5 to 9/group). * represents p < 0.05, and ** represents p < 0.01. K: Tumor volumes of mice (n = 5 to 6/group). Mice were intraperitoneally injected with 35 µg of αPD-L1 antibody or isotype control (isotype IgG) once weekly on days 9 and 16 for NC and days 11 and 18 for B2M KO #2. * represents p < 0.05. All data are shown as the mean ± SEM. Experiments were performed at least twice for A to F, or once for others. Statistical significance was determined using unpaired Student's t-test for (H), (J), and (K) and one-way ANOVA followed by Bonferroni's multiple comparisons test for (D) and (F).

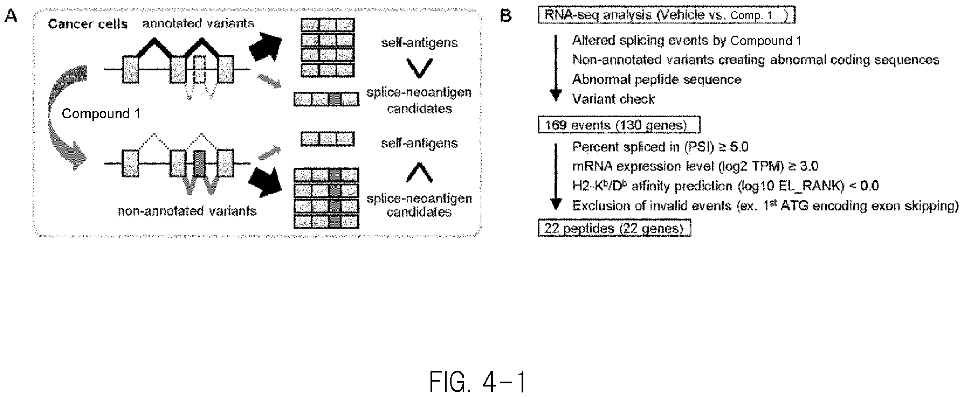

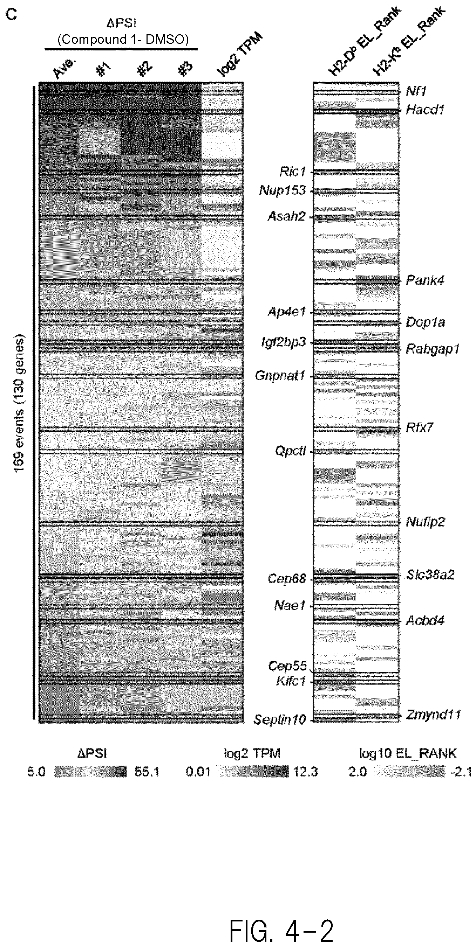

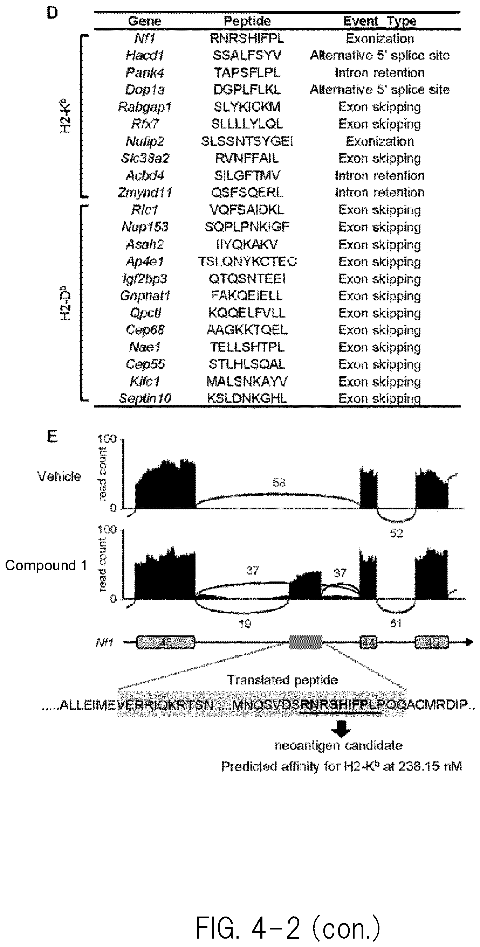

[FIG. 4-1] FIG. 4. Identification of Compound 1-inducible splice-neoantigen candidates using RNA-seq analysis. A: A diagram of enhanced tumor immunity through compound 1-induced splice-neoantigens. B: A scheme for the screening of Compound 1-induced splice-neoantigen candidates through transcriptome analysis and prediction for MHC class I presentation. RNA-seq was conducted for MC38 cells treated with 10 pM Compound 1 or 0.1% DMSO for 6 hours in three biological replicates each. RNA-seq analysis was performed as detailed in the "Experimental Methods" section.
[FIG. 4-2] Continuation of FIG. 4. C: A heatmap of Compound 1-induced 169 splicing events indicating ΔPSI (Compound 1-DMSO), log2 TPM (average of Compound 1-treated samples) and log10 EL_RANK for H2-D^{b} or H2-K^{b}. D: Alist of 22 splice-neoantigen candidates. E: An example of a selected splice-neoantigen candidate is indicated for Nf1.

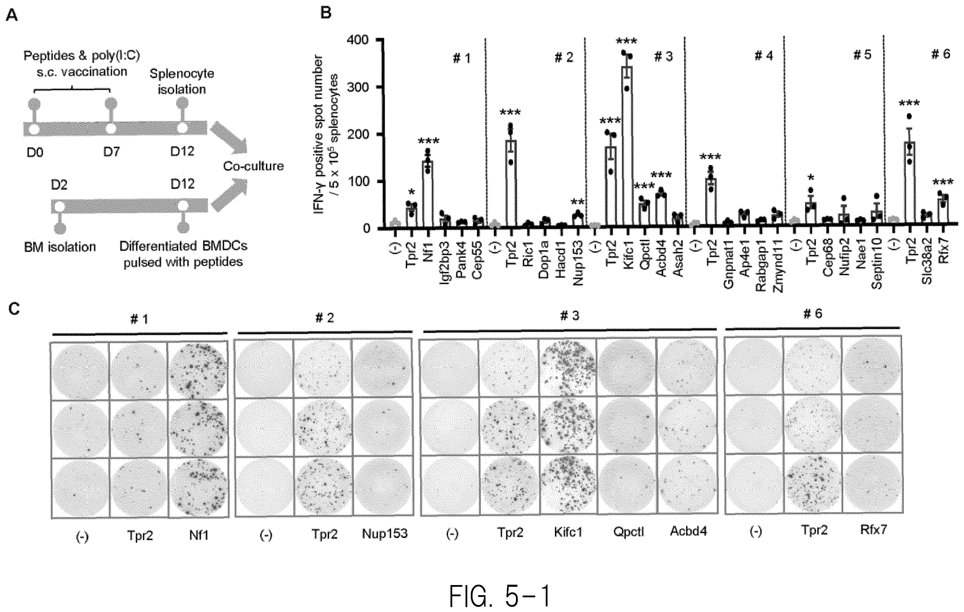

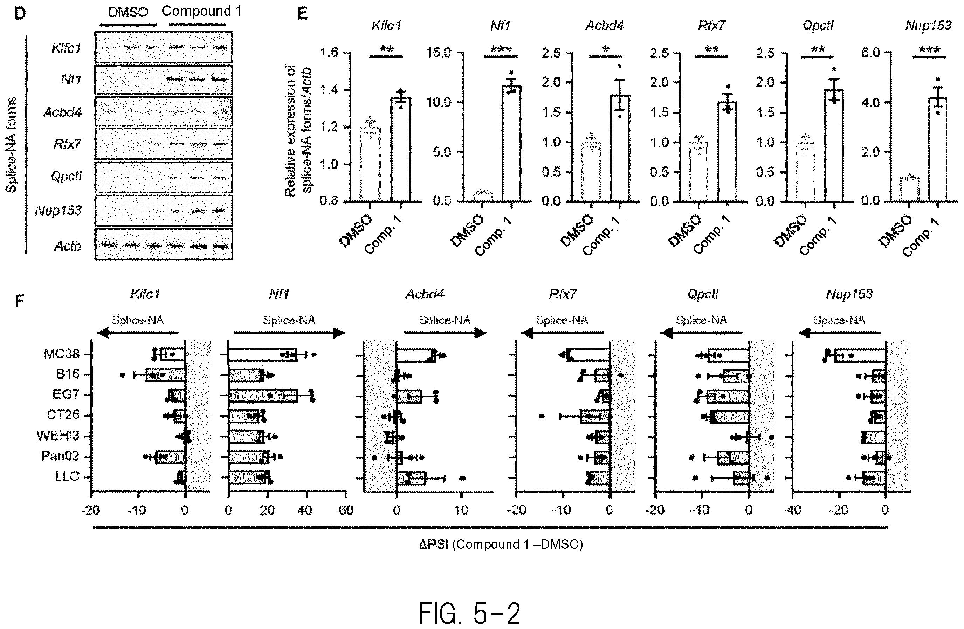

[FIG. 5-1] FIG. 5. Ex vivo validation of newly identified splice-neoantigen candidates. A: A schema for the ELISpot assay. B: IFN-γ ELISpot assay of Compound 1-induced splice-neoantigen candidates (n = 3/peptide). TRP2₁₈₀₋₁₈₈ peptide was used as a positive control. * represents p < 0.05, ** represents p < 0.01, and *** represents p < 0.001, vs. no peptide pulse shown as (-). C: ELISpot well images of the immunogenic peptides examined and corresponding positive and negative controls.
[FIG. 5-2] Continuation of FIG. 5. D and E: RT-PCR analysis for splice-neoantigen coding forms (splice- NA forms) in MC38 cells treated with 10 pM Compound 1 or vehicle control (0.1% DMSO) for 6 hours. Actb served as a loading control. * represents p < 0.05, ** represents p < 0.01, and *** represents p < 0.001. F: Splice-neoantigen producing rates calculated from RNA-seq data (n = 3 biological replicates) are indicated as the ΔPSI (Compound 1-DMSO). All data are shown as the mean ± SEM. All experiments were performed at least twice. Statistical significance was determined using unpaired Student's t-test for (B) and (E).

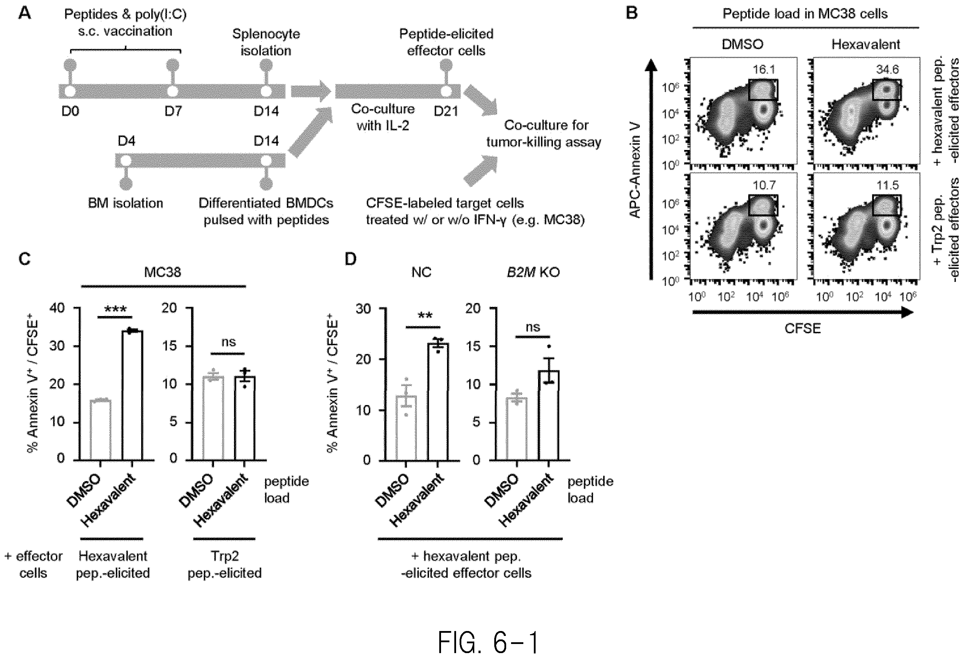

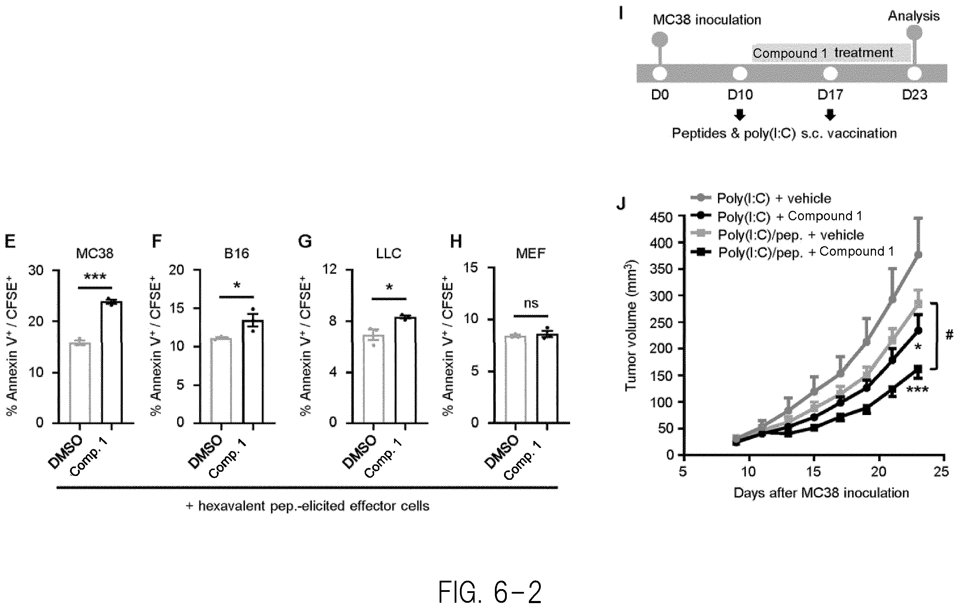

[FIG. 6-1] FIG. 6. Hexavalent vaccination of inducible splice-neoepitopes promotes Compound 1-induced cancer immune responses. A: A schema for the cancer-killing assay. B and C: Representative flow cytometric images showing tumor-killing activity mediated by antigen-elicited effector cells (B) and their quantitative data of annexin V⁺/CFSE⁺ frequency (C) are indicated (n = 3/group). Hexavalent (Kifc1-, Nf1-, Acbd4-, Rfx7-, Qpctl-, and Nup 153-derived) peptides or an irrelevant Trp2₁₈₀₋₁₈₈ peptide was used to elicit antigen-reactive effector cells. CFSE-labeled MC38 cells were stimulated with either 0.1% DMSO or hexavalent peptides for 2 hours and then co-cultured with effector cells for 24 hours. Annexin V was used for monitoring early and late apoptotic cell frequencies. *** represents p < 0.001. D: For the nontargeting control (NC) or B2M KO clone (n = 3/group), annexin V⁺/CFSE⁺ frequencies in the same experimental condition as (C) are shown. ** represents p < 0.01.
[FIG. 6-2] Continuation of FIG. 6. E to H: Effector cells elicited by hexavalent peptide-immunized mice were co-cultured with CFSE-labeled target cells in the presence of either 0.1% DMSO or 25 pM Compound 1 for 24 hours. Quantitative data of annexin V⁺/CFSE⁺ (n = 3/group) are shown for MC38 (E), B16 (F), LLC (G), and MEF (H). * represents p < 0.05, and*** represents p < 0.001. I: A schema for the vaccination study in MC38 tumor-bearing mice. J: Tumor volumes of mice (n = 8/group). * represents p < 0.05, and *** represents p < 0.01, vs. Poly(I:C) + vehicle. # represents p < 0.05, and ### represents p < 0.001, comparison between the indicated groups. All data are shown as the mean ± SEM. "ns" represents "not significant". All experiments were performed at least twice. Statistical significance was determined using unpaired Student's t-test for (C) to (H) and one-way ANOVA followed by Bonferroni's multiple-comparisons test for (J).

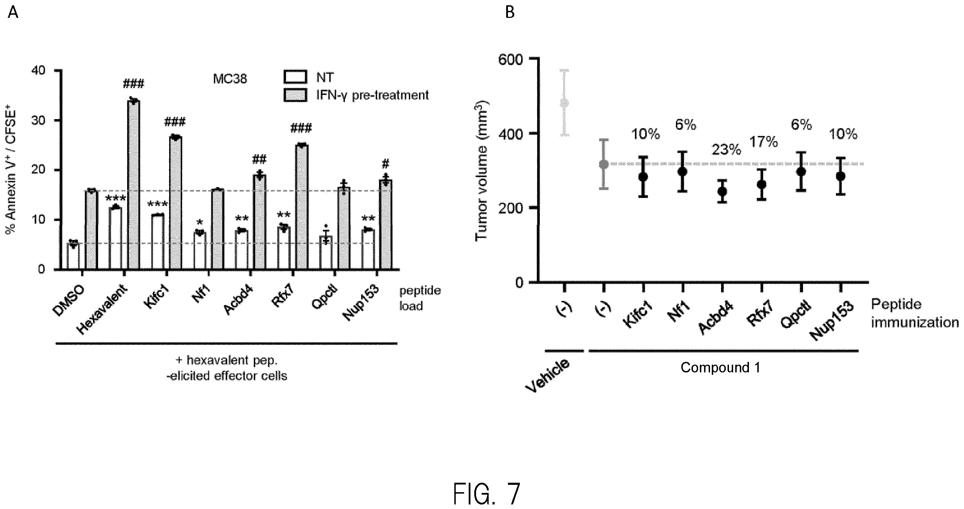

[FIG. 7] A: In vitro tumor-killing activity mediated by individual splice-neoantigens. Quantitative data of annexin V⁺/CFSE⁺ frequencies are shown (n = 3/group). CFSE-labeled MC38 cells, with or without IFN-pretreatment, were further stimulated with either 0.1% DMSO, hexavalent peptides, or individual peptides for 2 hours, and then co-cultured with hexavalent peptide-reactive effector cells for 24 hours. Data are shown as the mean ± SEM. Experiments were performed at least twice. B: Tumor suppressive effect by hexavalent or individual splice-neoantigen vaccination. Tumor volumes of mice on day 23 are shown. Mice were subcutaneously vaccinated with 100 µg of peptide and 50 µg of Poly(I:C) on days 11 and 18. In groups indicated by (-), 5% DMSO was injected instead of peptide. Compound 1 (10 pM) or vehicle control (0.1% DMSO) was intratumorally given once daily. Data are shown as the mean ± SEM (n = 7/group). Experiments were performed once.

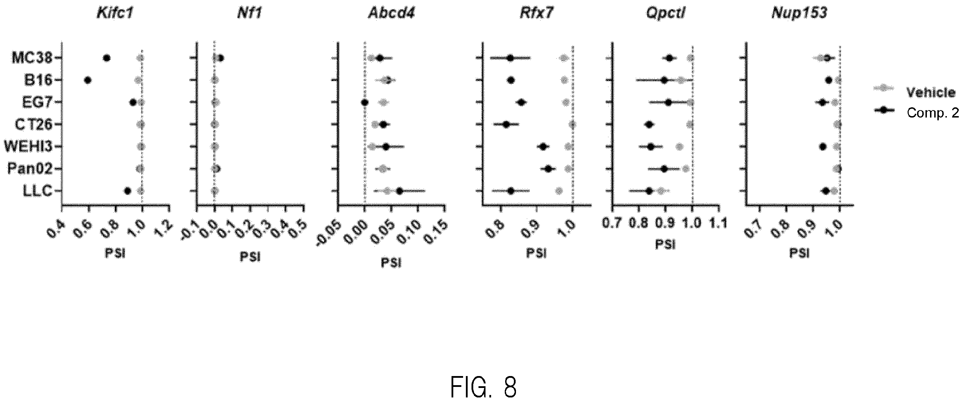

[FIG. 8] FIG. 8 shows RAN-Seq analysis results indicating that administration of Compound 2 caused splicing events (alterations in splicing) that produce splice-neoantigens in various cancer cells.

### Description of the Invention

In one aspect, the present disclosure is based on the finding that a splicing-controlling compound that modulates the activity of serine/arginine-rich splicing factors (SRSFs) can induce a different splice-neoantigen from those known and can improve tumor immunogenicity.

In one aspect, the present disclosure is based on the finding that a splicing-controlling compound that modulates the activity of SRSFs can enhance immune checkpoint therapy.

In one or more embodiments, a splicing-controlling compound of the present disclosure can alter the RNA splicing pattern in tumor cells to make the tumor cells more likely to be recognized by immune cells, thereby allowing immune checkpoint therapy to exert its efficacy even on malignant tumors for which immune checkpoint therapy has not been effective.

In one or more embodiments, a splicing-controlling compound of the present disclosure can selectively induce the production of splice-neoantigens in tumor cells rather than in cells other than tumor cells, for example, normal cells.

In one or more embodiments, a splicing-controlling compound of the present disclosure can induce the production of splice-neoantigens in a plurality of tumor cells across cancer types.

In one or more embodiments, a splicing-controlling compound of the present disclosure exerts its antitumor effects via CD8⁺ T cells and MHC class I of tumor cells and can therefore improve the PD-1 blocking effect and enhance immune checkpoint therapy.

As used in the present disclosure, the term "serine/arginine-rich splicing factors (SRSFs)" may collectively mean SR proteins involved in splicing.

As used in the present disclosure, the term "splicing' may mean RNA splicing unless otherwise mentioned.

As used in the present disclosure, the term "SR proteins" may mean proteins containing a protein domain (RS domain) with long repeat sequences of serine (S) and arginine (R). In one or more embodiments, SR proteins are proteins that are 200 to 600 amino acids in length and composed of two domains, an RNA recognition motif (RRM) region and an arginine/serine repeat (RS domain).

In the present disclosure, SRSFs are human or non-human animal SRSFs in one or more embodiments. SRSFs may include human SRSFs 1 to 12.

In one or more embodiments, the phrase "modulates the activity of SRSFs" as used in the present disclosure includes modulating the activity of at least one SRSF.

In one or more embodiments, the phrase "modulates the activity of SRSFs" as used in the present disclosure can include altering RNA splicing, or may include causing splicing events that produce splice-neoantigens to occur.

### "Splicing-controlling compound"

In one aspect, the term "splicing-controlling compound" as used in the present disclosure refers to a compound that can modulate the activity of SRSFs.

The splicing-controlling compound of the present disclosure, in one or more embodiments, may be a compound that can modulate a Cdc2-like kinase (CLK). Modulation of CLK may include suppressing or enhancing the kinase activity of CLK. In one or more embodiments, modulation of CLK may include modulating the activity of SRSFs through modulation of CLK.

The splicing-controlling compound of the present disclosure, in one or more embodiments, may be a compound that enhances the production of a peptide that improves tumor immunogenicity in a tumor.

As used in the present disclosure, the phrase "enhances the production of a peptide that improves tumor immunogenicity in a tumor" may mean increasing the expression level of abnormally spliced mRNA (mature mRNA) that may be translated to a "peptide that improves tumor immunogenicity".

The splicing-controlling compound of the present disclosure, in one or more embodiments, may be a compound that enhances (increases) the production of peptides (1) to (6), which will be described later, in the murine cancer cell line MC38.

The splicing-controlling compound of the present disclosure, in one or more embodiments, may not be a compound that modulates the splicing factor RBM39, or may not contain this compound. The splicing-controlling compound of the present disclosure, in one or more embodiments, is not Indisulam or MS-023 and does not contain either of them.

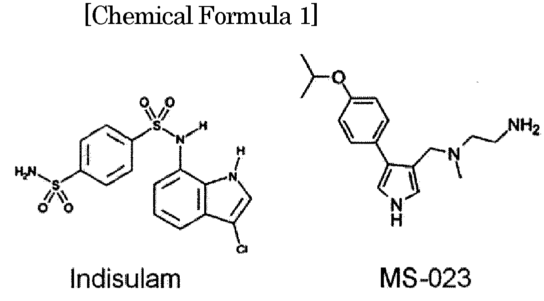

The splicing-controlling compound of the present disclosure, in one or more embodiments, may be at least one compound selected from the group consisting of compounds represented by the formulae (I) to (II) below and pharmaceutically acceptable salts thereof 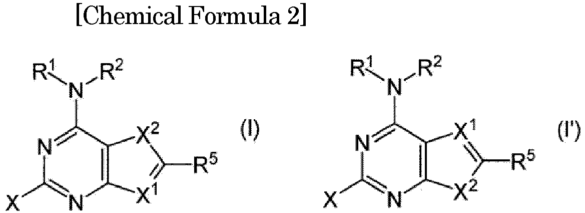 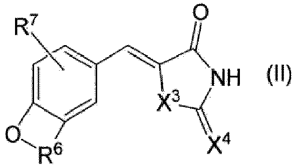 where, in the formulae (I) and (I'),
R¹ and R² each independently represent a hydrogen atom, a substituted or unsubstituted C₁-C₆ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, R¹ and R² are bonded to each other to form a ring together with N, and the ring is a substituted or unsubstituted monocyclic heterocyclic ring or a substituted or unsubstituted bicyclic heterocyclic ring,
R⁵ represents a hydrogen atom, a halogen atom, a substituted or unsubstituted C₁-C₆ alkoxy group, or a dialkylamino group;
X¹ represents N or -CH-;
X² represents -N(R³)-, S, or O;
R³ represents a hydrogen atom, a C₁-C₆ alkyl group, a benzyl or heteroarylmethyl group, a substituted or unsubstituted aryl group, a substituted or unsubstituted heteroaryl group, or CH₂OC(O)R⁴-;
R⁴ represents a C₁-C₆ alkyl group, a benzyl or heteroarylmethyl group, a substituted or unsubstituted aryl group, or a substituted or unsubstituted heteroaryl group; and
X represents a hydrogen atom, a halogen atom, an amino group, an amino group substituted with R¹ and R², an azido group, a cyano group, a nitro group, a hydroxy group, a C₁-C₆ alkyloxy group, a substituted or unsubstituted aryloxy group, a substituted or unsubstituted heteroaryloxy group, a mercapto group, a C₁-C₆ alkylthio group, a substituted or unsubstituted arylthio group, a substituted or unsubstituted heteroarylthio group, a benzyl or heteroarylmethyl group, a substituted or unsubstituted aryl group, or a substituted or unsubstituted heteroaryl group, and
in the formula (II),
X³ and X⁴ each independently represent S or NH;
R⁶ represents 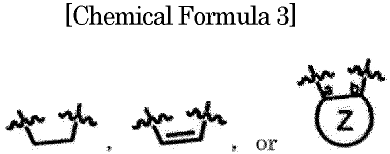
where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may have one or more substituents, the substituents being hydrogen, a halogen atom, or a C₁-C₆ alkyl group; and
R⁷ represents a hydrogen atom, a halogen atom, or a C₁-C₆ alkyl group.

In R⁶, the bonds indicated by the wavy lines are binding portions that bind to a compound represented by the formula (II).

In the present disclosure, the substituent intended by the phrase "substituted or unsubstituted" may be a single substituent or a plurality of identical or different substituents, and in one or more embodiments, examples thereof include a halogen atom, a cyano group, a trifluoromethyl group, a nitro group, a hydroxy group, a methylenedioxy group, a lower alkyl group, a lower alkoxy group, a benzyloxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, and a lower alkylsulfonamide group. In one or more embodiments, the halogen atom may be a fluorine atom, a chloride atom, a bromine atom, or an iodine atom.

In the formula (I) or (I'), when X¹ and X² represent N and NH, respectively, the formulae (I) and (I') are tautomers. Some of the specific examples above may illustrate only one of the tautomers, but in the present disclosure, it should be construed that disclosure of one of the tautomers also discloses the other tautomer. In the compound represented by the formula (I) or (I'), when a chiral carbon atom is present, and/or stereoisomers are present, the compound is a mixture of the isomers or an isolated isomer in one or more embodiments.

In the formula (I) or (I'), in one or more embodiments, R¹ may represent a heteroarylmethyl group, R² may represent a hydrogen atom, X¹ may represent N or - CH-, X² may represent -N(R³)-, S, or O, R³ may represent a hydrogen atom or a methyl group, R⁵ may represent a hydrogen atom, a halogen-substituted or unsubstituted C₁-C₆ alkoxy group, or a dialkylamino group, and X may represent a halogen atom.

In the formula (I) or (I'), in one or more embodiments, R¹ may represent a heteroarylmethyl group, R² may represent a hydrogen atom, X¹ may represent N or - CH-, X² may represent -NH-, S, or O, R⁵ may represent a hydrogen atom, and X may represent a halogen atom.

In the formula (I) or (I'), in one or more embodiments, R¹ may represent an oxygen-containing heteroarylmethyl group, R² may represent a hydrogen atom, X¹ may represent N, X² may represent -NH-, R⁵ may represent a hydrogen atom, and X may represent a halogen atom.

The oxygen-containing heteroarylmethyl group may be a furanylmethyl group.

In one or more embodiments, examples of the compound represented by the formula (I) or (I') include the following compounds.

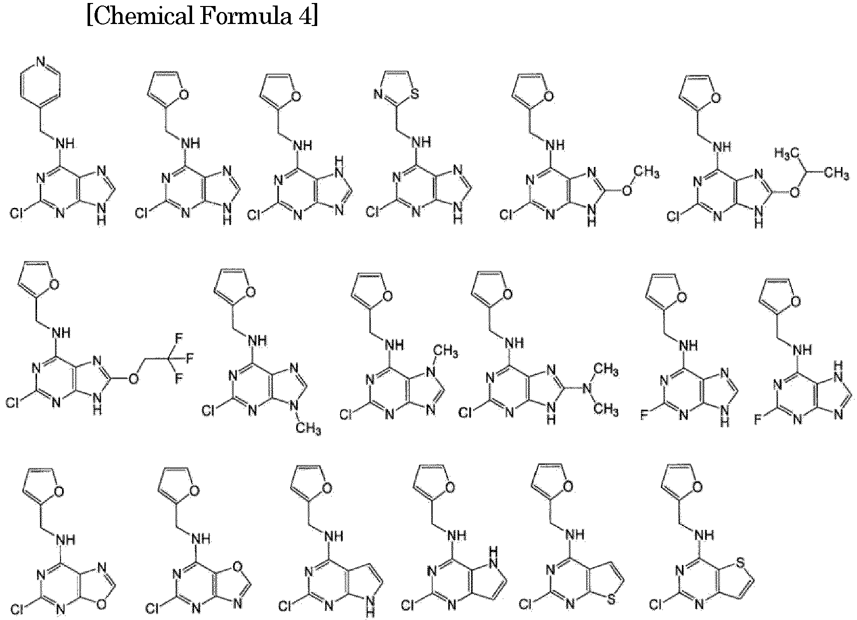

In the formula (II), in one or more embodiments, X³ and X⁴ each independently represent S or NH, R⁶ represents -CH₂-CH₂- or -CH=CH-, and R⁷ represents a hydrogen atom, a halogen atom, or a C₁-C₆ alkyl group.

In the formula (II), in one or more embodiments, X³ represents S, X⁴ represents S or NH, R⁶ represents -CH₂-CH₂- or -CH=CH-, and R⁷ represents a hydrogen atom or a halogen atom.

In one or more embodiments, examples of the compound represented by the formula (II) include the following compounds: 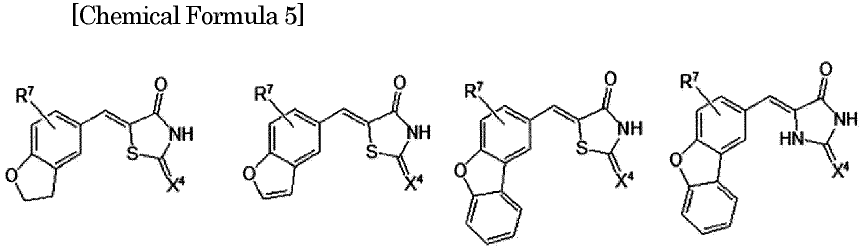 where R⁷ represents a hydrogen atom, a halogen atom, or a C₁-C₆ alkyl group, preferably a hydrogen atom, and X⁴ represents S or NH.

The splicing-controlling compound of the present disclosure, in one or more embodiments, may be at least one compound selected from the group consisting of compounds represented by the formulae (III) to (VII) below and pharmaceutically acceptable salts thereof.

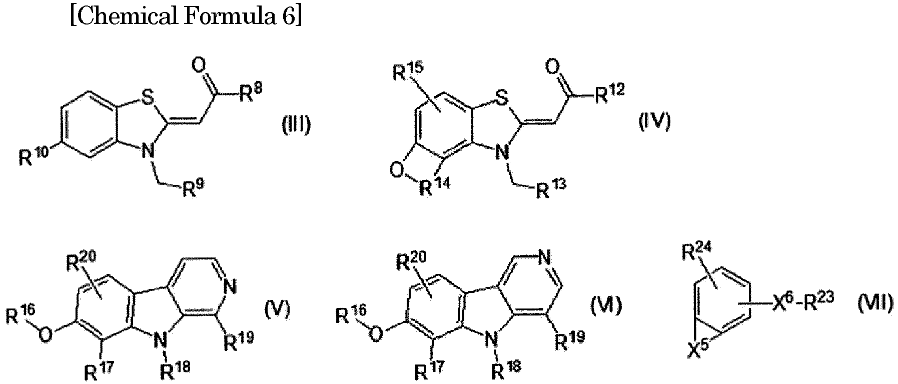

In the formula (III),
R⁸ and R⁹ each independently represent a hydrogen atom, a halogen-substituted or unsubstituted C₁-C₁₀ alkyl group, or a C₂-C₆ alkenyl group;
R¹⁰ represents a hydrogen atom, a halogen atom, or a halogen-substituted or unsubstituted C₁-C₁₀ alkyl group, -OR¹¹, -NHR¹¹, or -N(R¹¹)₂; and
R¹¹ represents a hydrogen atom or a C₁-C₁₀ alkyl group.

In one or more embodiments, examples of the compound represented by the formula (III) include the following compounds: 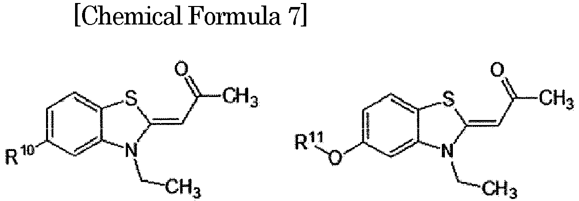 where R¹⁰ represents a hydrogen atom, a halogen atom, or a halogen-substituted or unsubstituted C₁-C₁₀ alkyl group, preferably a hydrogen atom, a fluorine atom, a chlorine atom, a methyl group, or an ethyl group, and R¹¹ represents a hydrogen atom or a C₁-C₁₀ alkyl group, preferably a hydrogen atom, a methyl group, or an ethyl group.

In one or more embodiments, examples of the compound represented by the formula (III) include the following compounds.

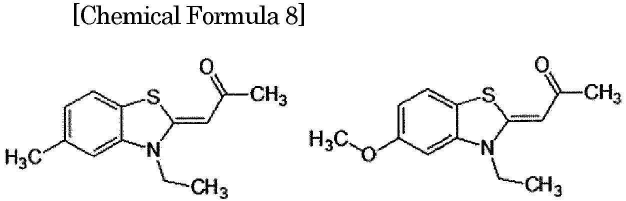

In the formula (IV),
R¹² and R¹³ each independently represent a hydrogen atom or a C₁-C₆ alkyl group;
R¹⁴ represents 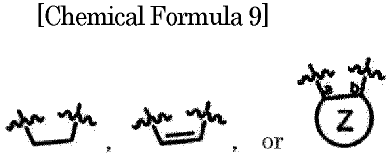
where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may have one or more substituents, the substituents being hydrogen, a halogen atom, or a C₁-C₆ alkyl group; and
R¹⁵ represents a hydrogen atom, a halogen atom, or a C₁-C₆ alkyl group.

In R¹⁴, the bonds indicated by the wavy lines are binding portions that bind to a compound represented by the formula (IV).

In one or more embodiments, examples of the compound represented by the formula (IV) include the following compounds: 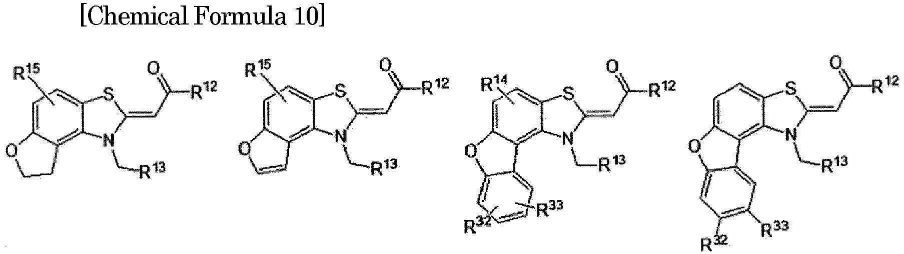 where R¹² and R¹³ each independently represent a hydrogen atom or a C₁-C₆ alkyl group, preferably a hydrogen atom, a methyl group, or an ethyl group, and more preferably a methyl group, R¹⁵ represents a hydrogen atom, a halogen atom, or a C₁-C₆ alkyl group, preferably a hydrogen atom, a fluorine atom, a chlorine atom, or a methyl group, and R³² and R³³ each independently represent a hydrogen atom, a halogen atom, or a C₁-C₆ alkyl group, preferably a hydrogen atom, a fluorine atom, a chlorine atom, or a methyl group, and, more preferably, one of R³² and R³³ represents a hydrogen atom and the other represents a chlorine atom or a methyl group, or both R³² and R³³ represent methyl groups.

In one or more embodiments, examples of the compound represented by the formula (IV) include the following compounds: 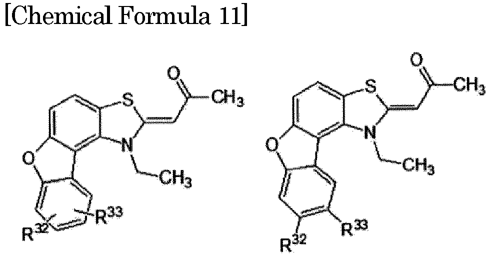 where R³² and R³³ each independently represent a hydrogen atom, a halogen atom, or a C₁-C₆ alkyl group, preferably a hydrogen atom, a fluorine atom, a chlorine atom, or a methyl group, and, more preferably, one of R³² and R³³ represents a hydrogen atom and the other represents a chlorine atom or a methyl group, or both R³² and R³³ represent methyl groups.

In one or more embodiments, examples of the compound represented by the formula (IV) include the following compounds.

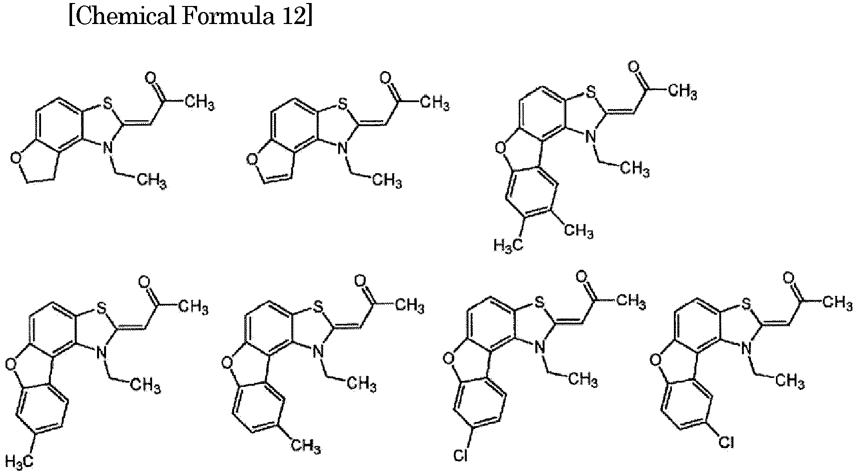

In the formulae (V) and (VI),
R¹⁶ and R¹⁸ each independently represent a hydrogen atom, a C₁-C₆ alkyl group, a benzyl or heteroarylmethyl group, a substituted or unsubstituted aryl group, or a substituted or unsubstituted heteroaryl group;
R¹⁷ represents -R²¹, -C≡C-R²¹, -CH=CH-R²¹, or -O-(CH₂)n-R²¹, where n represents 1 to 6, and R²¹ represents a hydrogen atom, a hydroxy group, a C₁-C₈ alkyl group, -Si(R²²)₃, or a substituted or unsubstituted phenyl group, a monocyclic heteroaromatic ring group, or a cycloaliphatic group;
alternatively, R¹⁶ and R¹⁷ are bonded to each other to form a ring, and -R¹⁶-R¹⁷-represents -(CH₂)ₘ-CH₂-, -CH=CH-, -(CH₂)ₘ-O-, or any of these substituted with a halogen atom, where m represents 1 to 6, R²² represents a hydrogen atom, a C₁-C₆ alkyl group, a trihalomethyl group, or a hydroxy group, and the three atoms or groups R²² in -Si(R²²)₃ may be different from one another; and
R¹⁹ and R²⁰ represent hydrogen atoms or C₁-C₆ alkyl groups.

In one or more embodiments, examples of the compound represented by the formula (V) or (VI) include the following compounds: 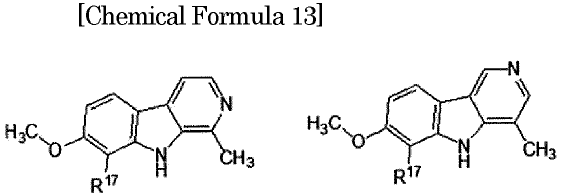 where R¹⁷ represents a hydrogen atom, a hydroxy group, or a C₁-C₆ alkyl group, preferably a hydrogen atom, a hydroxy group, or a methyl group.

In one or more embodiments, examples of the compound represented by the formula (V) or (VI) include the following compounds.

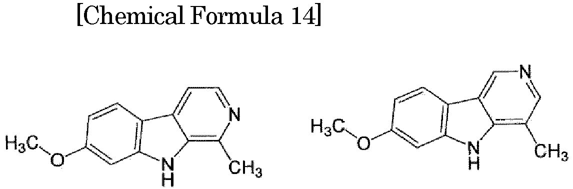

In the formula (VII),
X⁵ represents 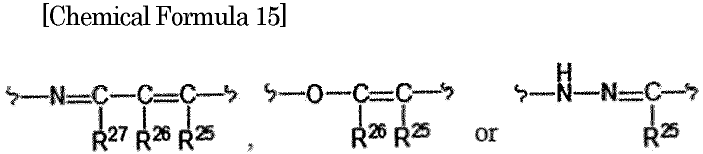
where R²⁵, R²⁶, and R²⁷ each independently represent hydrogen, a halogen atom, a carboxyl group, an amino group, a hydroxy group, a C₁-C₄ alkyl group, or a C₁-C₄ alkyl group substituted with a halogen atom;
X⁶ represents -(bond) or -NH-;
R²³ represents 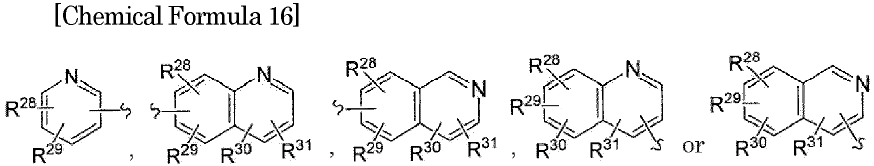
where R²⁸, R²⁹, R³⁰, and R³¹ each independently represent hydrogen, a halogen atom, a carboxyl group, an amino group, a hydroxy group, a C₁-C₄ alkyl group, or a C₁-C₄ alkyl group substituted with a halogen atom; and
R²⁴ represents a hydrogen atom, a halogen atom, a carboxyl group, an amino group, a hydroxy group, or a halogen-substituted or unsubstituted C₁-C₄ alkyl group.

In X⁵ and R²³, the bonds indicated by the wavy lines are binding portions that bind to a compound represented by the formula (VII).

In one or more embodiments, examples of the compound represented by the formula (VIII) include the following compounds: 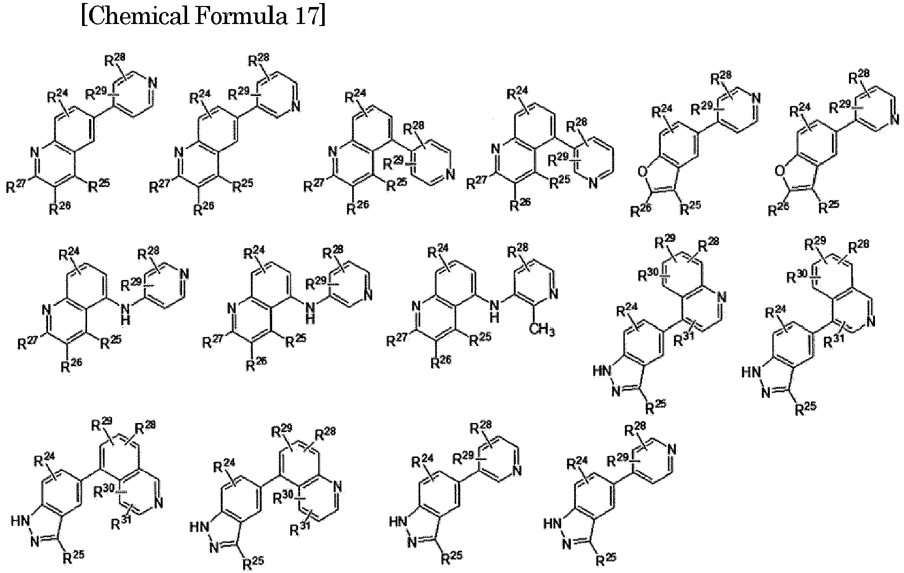 where R²⁴, R²⁵, R²⁶, R²⁷, R²⁸, R²⁹, R³⁰, and R³¹ each independently represent a hydrogen atom, a halogen atom, a carboxyl group, an amino group, a hydroxy group, a C₁-C₄ alkyl group, or a C₁-C₄ alkyl group substituted with a halogen atom, preferably a hydrogen atom, a fluorine atom, a chlorine atom, or a methyl group.

In one or more embodiments, examples of the compound represented by the formula (VII) include the following compounds.

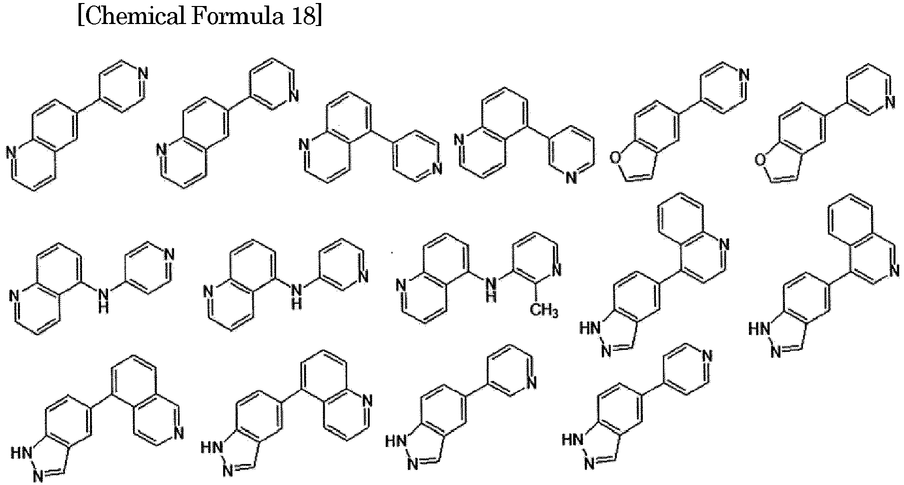

The term "pharmaceutically acceptable salts" as used in the present disclosure includes pharmaceutically, pharmacologically, and/or medicinally acceptable salts, and examples thereof include inorganic acid salts, organic acid salts, inorganic base salts, organic base salts, acidic or basic amino acid salts, and the like.

Preferred examples of the inorganic acid salts include hydrochloride, hydrobromide, sulfate, nitrate, phosphate, and the like.

Preferred examples of the organic acid salts include acetate, succinate, fumarate, maleate, tartrate, citrate, lactate, stearate, benzoate, methanesulfonate, p-toluenesulfonate, and the like.

Preferred examples of the inorganic base salts include alkali metal salts such as sodium and potassium salts, alkaline earth metal salts such as calcium and magnesium salts, aluminum salts, ammonium salts, and the like.

Preferred examples of the organic base salts include diethylamine salts, diethylamine salts, meglumine salts, N,N'-dibenzylethylenediamine salts, and the like.

Preferred examples of the acidic amino acid salts include aspartate, glutamate, and the like.

Preferred examples of the basic amino acid salts include arginine salts, lysine salts, omithine salts, and the like.

In the present disclosure, a "salt of a compound" may include a hydrate that can be formed as a result of a compound being left to stand in the atmosphere and thereby absorbing moisture. In the present disclosure, a "salt of a compound" may also include a solvate that can be formed as a result of a compound absorbing a certain other type of solvent.

### <Peptide that improves tumor immunogenicity>

In one or more embodiments, the "peptide that improves tumor immunogenicity" of the present disclosure is a splice-neoantigen.

In the present disclosure, the splice-neoantigen may mean, in one or more embodiments, a cancer-specific antigen produced through translation of mRNA generated by abnormal splicing. In the present disclosure, the splice-neoantigen is, in one or more embodiments, a splice-neoantigen produced by bringing the splicing-controlling compound of the present disclosure into contact with a tumor.

In the present disclosure, the splice-neoantigen may mean, in one or more embodiments, an antigen that can be presented by MHC class I of tumor cells.

Therefore, in one aspect, the present disclosure relates to a method for producing a splice-neoantigen, the method including bringing a tumor and a splicing-controlling compound of the present disclosure into contact with each other.

The peptide (splice-neoantigen) that improves tumor immunogenicity of the present disclosure may be, in one or more embodiments, any one of the peptides (1) to (6) below and a combination of two, three, four, five, or six of these peptides.

Peptide (1): A peptide containing the amino acid sequence MALSNKAYV (SEQ ID No: 1), or a peptide in which one to several amino acids of an amino acid sequence corresponding to the sequence of SEQ ID No: 1 in the above peptide are deleted, substituted, and/or added and which has the ability to improve tumor immunogenicity.

Peptide (2): A peptide containing the amino acid sequence RNRSHIFPL (SEQ ID No: 2), or a peptide in which one to several amino acids of an amino acid sequence corresponding to the sequence of SEQ ID No: 2 in the above peptide are deleted, substituted, and/or added and which has the ability to improve tumor immunogenicity.

Peptide (3): A peptide containing the amino acid sequence SILGFTMV (SEQ ID No: 3), or a peptide in which one to several amino acids of an amino acid sequence corresponding to the sequence of SEQ ID No: 3 in the above peptide are deleted, substituted, and/or added and which has the ability to improve tumor immunogenicity.

Peptide (4): A peptide containing the amino acid sequence SLLLLYLQL (SEQ ID No: 4), or a peptide in which one to several amino acids of an amino acid sequence corresponding to the sequence of SEQ ID No: 4 in the above peptide are deleted, substituted, and/or added and which has the ability to improve tumor immunogenicity.

Peptide (5): A peptide containing the amino acid sequence KQQELFVLL (SEQ ID No: 5), or a peptide in which one to several amino acids of an amino acid sequence corresponding to the sequence of SEQ ID No: 5 in the above peptide are deleted, substituted, and/or added and which has the ability to improve tumor immunogenicity.

Peptide (6): A peptide containing the amino acid sequence SQPLPNKIGF (SEQ ID No: 6), or a peptide in which one to several amino acids of an amino acid sequence corresponding to the sequence of SEQ ID No: 6 in the above peptide are deleted, substituted, and/or added and which has the ability to improve tumor immunogenicity.

In the peptides (1) to (6), the term "several" may mean two or three, for example.

In one or more embodiments, the peptide that improves tumor immunogenicity of the present disclosure may be at least one of the peptides (1) and (2).

In the present disclosure, a "peptide containing (comprising) an amino acid sequence" may be a peptide with an additional amino acid added to the N-terminal and/or the C-terminal of the amino acid sequence, or may be a peptide "consisting of the amino acid sequence" with no additional amino acid added. Since the amino acid sequences corresponding to the sequence of SEQ ID Nos: 1 to 6 are translation sites resulting from splicing abnormalities caused by the splicing-controlling compound of the present disclosure, the above-described peptides (1) to (6) are usually translated while containing an amino acid sequence translated from other exons, and then function as neoantigens containing or consisting of amino acid sequences corresponding to the sequence of SEQ ID Nos: 1 to 6.

In one or more embodiments, the phrase "improves tumor immunogenicity" as used in the present disclosure may include suppressing tumor growth.

In one or more embodiments, the phrase "improves tumor immunogenicity" as used in the present disclosure may include increasing infiltration of CD8⁺ T cells into a tumor site.

In one or more embodiments, the phrase "improves tumor immunogenicity" as used in the present disclosure may include enhancing the antitumor effects of an immune checkpoint inhibitor.

In one or more embodiments, the phrase "improves tumor immunogenicity" as used in the present disclosure may include cancer immunotherapy.

In one or more embodiments, the phrase "improves tumor immunogenicity" as used in the present disclosure may include improvement, inhibition of progression, and/or treatment of cancer.

In one or more embodiments, the phrase "improves tumor immunogenicity" as used in the present disclosure may include enhancing immune checkpoint therapy.

In one or more embodiments, the term "immune checkpoint therapy" as used in the present disclosure may mean cancer immunotherapy using an immune checkpoint inhibitor.

In the present disclosure, "tumor" may be, in one or more embodiments, a malignant tumor. In the present disclosure, "malignant tumor" may have the same meaning as "cancer".

In the present disclosure, "cancer" may include leukemia, multiple myeloma, and lymphoma. Also, "cancer" may include primary or recurrent cancers. Non-limiting examples of cancer include lung cancer, non-small-cell lung cancer (NSCLC), small cell lung cancer (SCLC), digestive cancer, gastrointestinal cancer, colorectal cancer, gastrointestinal stromal tumor, gastrointestinal carcinoid tumor, colon cancer, rectal cancer, anal cancer, bile duct cancer, small intestine cancer, gastric cancer, esophageal cancer, gallbladder cancer, liver cancer; pancreatic cancer, appendiceal cancer, breast cancer, ovarian cancer, renal cancer, renal cell cancer, prostate cancer, central nervous system cancer, skin cancer, melanoma, lymphoma, glioblastoma, mesothelioma, choriocarcinoma, cholangiocarcinoma, alveolar soft part sarcoma, head and neck cancer, thyroid cancer, osteogenic sarcoma, and blood cancer. These cancers or tumors may be, in one or more embodiments, cancers or tumors in mammals such as humans, non-human primates, rodents, mice, dogs, cats, horses, cows, sheep, pigs, goats, camels, and antelopes.

In the present disclosure, the subject whose tumor immunogenicity is to be improved, the subject of cancer immunotherapy, and the subject of improvement, inhibition of progression, and/or treatment of cancer may be, in one or more embodiments, individuals in need of these treatments, and the individuals may include, in one or more embodiments, mammals such as humans, non-human primates, rodents, mice, dogs, cats, horses, cows, sheep, pigs, goats, camels, and antelopes.

In the present disclosure, the immune checkpoint inhibitor may be, in one or more embodiments, an agent against a molecule selected from the group consisting of (1) CTLA-4, (2) PD-1, (3) LAG-3, (4) BTLA, (5) KIR, (6) TIM-3, (7) PD-L1, (8) PD-L2, (9) B7-H3, (10) B7-H4, (11) HVEM, (12) GAL9, (13) CD160, (14) VISTA, (15) BTNL2, (16) TIGIT, (17) PVR, (18) BTN1A1, (19) BTN2A2, (20) BTN3A2, and (21) CSF-1R and combinations thereof. In one or more embodiments, examples of the agent include an antibody, a partial antibody, a low-molecular-weight antibody, and a low-molecular-weight compound. In one or more embodiments, the immune checkpoint inhibitor maybe an anti-PD-1 monoclonal antibody, an anti-PD-L1 monoclonal antibody, or an anti-PD-L2 monoclonal antibody.

### <Pharmaceutical composition>

In one aspect, the present disclosure relates to a pharmaceutical composition (hereinafter also referred to as "pharmaceutical composition of the present disclosure") containing the splicing-controlling compound of the present disclosure as an active ingredient. The splicing-controlling compound of the present disclosure is as described above.

In one or more embodiments, the pharmaceutical composition of the present disclosure may be a pharmaceutical composition containing, as an active ingredient, a splicing-controlling compound that modulates the activity of SRSFs.

In one or more embodiments, the pharmaceutical composition of the present disclosure may be a pharmaceutical composition containing, as an active ingredient, a compound that can modulate a Cdc2-like kinase (CLK).

In one or more embodiments, the pharmaceutical composition of the present disclosure may be a pharmaceutical composition containing, as an active ingredient, a compound that causes a tumor to increase the production of a peptide that improves tumor immunogenicity. The peptide that improves tumor immunogenicity is as described above.

In one or more embodiments, the pharmaceutical composition of the present disclosure may be a pharmaceutical composition containing, as an active ingredient, a compound that causes a tumor to increase the production of a peptide selected from the group consisting of the above-described peptides (1) to (6) and combinations of two to six of these peptides. In one or more further embodiments, the pharmaceutical composition of the present disclosure may be a pharmaceutical composition containing, as an active ingredient, a compound that causes a tumor to increase the production of at least one of the peptides (1) and (2).

The splicing-controlling compound, which is an active ingredient of the pharmaceutical composition of the present disclosure, in one or more embodiments, may not contain a compound that modulates the splicing factor RBM39, as an active ingredient.

In addition, the splicing-controlling compound, which is an active ingredient of the pharmaceutical composition of the present disclosure, in one or more embodiments, may not contain Indisulam and MS-023 as active ingredients.

In one or more embodiments, the pharmaceutical composition of the present disclosure contains, as an active ingredient, a compound represented by the formula (I), (I'), (II), (III), (IV), (V), (VI), or (VII), or a combination thereof, and may further contain a medicinally acceptable carrier, antiseptic, diluent, or excipient, or other medicinally acceptable components.

In one or more embodiments, the pharmaceutical composition of the present disclosure is a pharmaceutical composition for use in improving tumor immunogenicity.

In one or more embodiments, the pharmaceutical composition of the present disclosure is a pharmaceutical composition for use in cancer immunotherapy.

In one or more embodiments, the pharmaceutical composition of the present disclosure is a pharmaceutical composition for use in improvement, inhibition of progression, and/or treatment of cancer.

In one or more embodiments, the pharmaceutical composition of the present disclosure is a pharmaceutical composition for use in enhancing immune checkpoint therapy.

In one or more embodiments, the pharmaceutical composition of the present disclosure is a pharmaceutical composition for use in combination with an immune checkpoint inhibitor. In one or more embodiments, the combined use may include simultaneously, separately, or sequentially administering the pharmaceutical composition and the immune checkpoint inhibitor in combination.

In one or more embodiments, the "pharmaceutical composition" of the present disclosure can be made into a dosage form suitable for the form of administration by applying a well-known drug preparation technique. Examples of the form of administration include, but are not limited to, oral administration using dosage forms such as tablets, capsules, granules, powders, pills, troches, syrups, or liquids. Other examples include parenteral administration using dosage forms such as injectables, liquid formulations, aerosols, suppositories, patches, poultices, lotions, liniments, ointments, or ophthalmic solutions. The pharmaceutical composition of the present disclosure may be produced in a well-known manner using additives such as, but not limited to, an excipient, a lubricant, a binder, a disintegrant, a stabilizing agent, a corrigent, and a diluent.

In one or more embodiments, the pharmaceutical composition according to the present disclosure may not contain other active ingredients having therapeutic effects, or may contain one or more additional active ingredients.

Examples of the excipient include, but are not limited to, starches such as starch, potato starch, and corn starch, lactose, crystalline cellulose, and calcium hydrogen phosphate.

Examples of the lubricant include, but are not limited to, ethyl cellulose, hydroxypropyl cellulose, hydroxypropyl methylcellulose, shellac, talc, carnauba wax, and paraffin.

Examples of the binder include, but are not limited to, polyvinylpyrrolidone, macrogol, and compounds that are similar to those given as examples of the excipient.

Examples of the disintegrant include, but are not limited to, compounds that are similar to those given as examples of the excipient and chemically-modified starches and celluloses such as croscarmellose sodium, sodium carboxymethyl starch, and crosslinked polyvinylpyrrolidone.

Examples of the stabilizing agent include, but are not limited to, para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid.

Examples of the corrigent include, but are not limited to, sweeteners, acidulants, and flavors that are often used.

To produce liquid formulations, ethanol, phenol, chlorocresol, purified water, distilled water, or the like can be used, without limitation, as a solvent, and a surfactant, an emulsifying agent, or the like can also be used as needed. Examples of the surfactant or the emulsifying agent include, but are not limited to, polysorbate 80, polyoxyl 40 stearate, and lauromacrogol.

Methods of use of the pharmaceutical composition according to the present disclosure may vary depending on the symptoms, age, administration method, and the like. Examples of the method of use include, but are not limited to, the following methods: an effective amount of the splicing-controlling compound of the present disclosure, which is an active ingredient, can be intermittently or continuously administered orally, percutaneously, submucosally, subcutaneously, intramuscularly, intravascularly, intracerebrally, or intraperitoneally, or the pharmaceutical composition can be intermittently or continuously administered orally, percutaneously, submucosally, subcutaneously, intramuscularly, intravascularly, intracerebrally, or intraperitoneally so as to achieve a concentration of the splicing-controlling compound of the present disclosure, which is an active ingredient, between 100 nM and 1 mM in the body. In a non-limiting embodiment, in the case of oral administration, the pharmaceutical composition may be administered to a subject (an adult human if the subject is a human) in an amount ranging from a lower limit of 0.01 mg (preferably 0.1 mg) to an upper limit of 2000 mg (preferably 500 mg, or more preferably 100 mg), in terms of the amount of the splicing-controlling compound of the present disclosure, per day in one or several doses, according to the symptoms. In a non-limiting embodiment, in the case of intravenous administration, the pharmaceutical composition may be administered to a subject (an adult human if the subject is a human) in an amount ranging from a lower limit of 0.001 mg (preferably 0.01 mg) to an upper limit of 500 mg (preferably 50 mg) per day in one or several doses, according to the symptoms.

In the present disclosure, the "effective amount" may be, in one or more embodiments, an amount that can improve tumor immunogenicity, an amount that can suppress tumor growth, an amount that can increase infiltration of CD8⁺ T cells into a tumor site, an amount that enables improvement, inhibition of progression, and/or treatment of cancer, or an amount that can enhance immune checkpoint therapy.

### <Method>

In one aspect, the present disclosure relates to a method for improving tumor immunogenicity, the method including administering the pharmaceutical composition of the present disclosure to a subject.

The subject may be defined above, and the administration method and dosage may be as described above.

In one or more embodiments, administering the pharmaceutical composition of the present disclosure to a subject may include bringing the pharmaceutical composition of the present disclosure into contact with a tumor.

In one or more embodiments, the method of the present disclosure may be a method for improving tumor immunogenicity, the method including bringing the splicing-controlling compound of the present disclosure into contact with a tumor. In addition, in one or more embodiments, the method of the present disclosure may be a method for improving tumor immunogenicity, the method including bringing a splicing-controlling compound that modulates the activity of SRSFs into contact with a tumor.

In one or more embodiments, the method of the present disclosure may be a method of cancer immunotherapy, the method including administering the pharmaceutical composition of the present disclosure to a subject or bringing the present disclosure into contact with a tumor.

In one or more embodiments, the method of the present disclosure may be improvement, inhibition of progression, and/or treatment of cancer, including administering the pharmaceutical composition of the present disclosure to a subject or bringing the present disclosure into contact with a tumor.

In one or more embodiments, the method of the present disclosure may be a method for enhancing immune checkpoint therapy, the method including administering the pharmaceutical composition of the present disclosure to a subject or bringing the present disclosure into contact with a tumor.

In one or more embodiments, the method of the present disclosure may include combined use of the pharmaceutical composition of the present disclosure and an immune checkpoint inhibitor. In one or more embodiments, the combined use may include simultaneously, separately, or sequentially administering the pharmaceutical composition of the present disclosure and the immune checkpoint inhibitor in combination.

The method for administering the immune checkpoint inhibitor is not particularly limited, and may be similar to a method that is employed when the agent is used alone.

### <Use>

In one or more embodiments, the present disclosure relates to use of the pharmaceutical composition of the present disclosure to improve tumor immunogenicity.

In one or more embodiments, the use of the present disclosure may be use of the pharmaceutical composition of the present disclosure for cancer immunotherapy.

In one or more embodiments, the use of the present disclosure may be use of the pharmaceutical composition of the present disclosure for improvement, inhibition of progression, and/or treatment of cancer.

In one or more embodiments, the use of the present disclosure may be use of the pharmaceutical composition of the present disclosure to enhance immune checkpoint therapy.

In one or more embodiments, the use of the present disclosure may be use of the pharmaceutical composition of the present disclosure for use in combination with an immune checkpoint inhibitor.

In one or more embodiments, the present disclosure relates to use of a combination of the pharmaceutical composition of the present disclosure and an immune checkpoint inhibitor for cancer immunotherapy.

Specific methods of use of the pharmaceutical composition of the present disclosure may be as described above.

In other embodiments, the present disclosure relates to use of a splicing-controlling compound that modulates the activity of SRSFs to produce a pharmaceutical composition of the present disclosure.

### <Second pharmaceutical composition>

In other aspects, the present disclosure relates to a pharmaceutical composition (hereinafter also referred to as "second pharmaceutical composition of the present disclosure") containing, as an active ingredient, at least one selected from the group consisting of a splice-neoantigen produced by bringing the splicing-controlling compound of the present disclosure into contact with a tumor, the above-described peptides (1) to (6) and combinations of two to six of these peptides, and pharmaceutically acceptable salts thereof.

The splice-neoantigen produced by bringing the splicing-controlling compound of the present disclosure into contact with a tumor and/or the peptides (1) to (6), of the second pharmaceutical composition of the present disclosure, may function as a vaccine that improves tumor immunogenicity.

Therefore, in one or more embodiments, the second pharmaceutical composition of the present disclosure is a pharmaceutical composition for improving immunogenicity of a tumor, the pharmaceutical composition containing, as an active ingredient, a splice-neoantigen produced by the tumor in contact with the splicing-controlling compound of the present disclosure.

The second pharmaceutical composition of the present disclosure may be used in combination with the pharmaceutical composition of the present disclosure.

The second pharmaceutical composition of the present disclosure may be used in combination with an immune checkpoint inhibitor.

The second pharmaceutical composition of the present disclosure may be used in combination with the pharmaceutical composition of the present disclosure and an immune checkpoint inhibitor.

Uses and methods of use of the second pharmaceutical composition of the present disclosure can be similar to those of the pharmaceutical composition of the present disclosure.

### <Kit>

In one or more embodiments, the present disclosure relates to a kit for use in cancer immunotherapy, the kit including the pharmaceutical composition of the present disclosure and an immune checkpoint inhibitor.

The kit of the present disclosure may further include the second pharmaceutical composition of the present disclosure.

The present disclosure may relate to one or more embodiments below.
<1> A pharmaceutical composition for improving tumor immunogenicity, the composition containing, as an active ingredient, a splicing-controlling compound that modulates activity of serine/arginine-rich splicing factors (SRSFs).
<2> The pharmaceutical composition according to clause <1>, wherein the compound is a compound that can modulate a Cdc2-like kinase (CLK).
<3> The pharmaceutical composition according to clause <1> or <2>, wherein the compound is a compound that causes a tumor to increase production of a peptide that improves tumor immunogenicity.
<4> The pharmaceutical composition according to any one of clauses <1> to <3>, wherein the peptide is selected from the group consisting of the peptides (1) to (6) below and combinations of two to six of these peptides:
   peptide (1): a peptide containing the amino acid sequence MALSNKAYV (SEQ ID No: 1), or a peptide in which one to several amino acids of an amino acid sequence corresponding to the sequence of SEQ ID No: 1 in the above peptide are deleted, substituted, and/or added and which has the ability to improve tumor immunogenicity;
   peptide (2): a peptide containing the amino acid sequence RNRSHIFPL (SEQ ID No: 2), or a peptide in which one to several amino acids of an amino acid sequence corresponding to the sequence of SEQ ID No: 2 in the above peptide are deleted, substituted, and/or added and which has the ability to improve tumor immunogenicity;
   peptide (3): a peptide containing the amino acid sequence SILGFTMV (SEQ ID No: 3), or a peptide in which one to several amino acids of an amino acid sequence corresponding to the sequence of SEQ ID No: 3 in the above peptide are deleted, substituted, and/or added and which has the ability to improve tumor immunogenicity;
   peptide (4): a peptide containing the amino acid sequence SLLLLYI.ΓaL (SEQ ID No: 4), or a peptide in which one to several amino acids of an amino acid sequence corresponding to the sequence of SEQ ID No: 4 in the above peptide are deleted, substituted, and/or added and which has the ability to improve tumor immunogenicity;
   peptide (5): a peptide containing the amino acid sequence KQQELFVLL (SEQ ID No: 5), or a peptide in which one to several amino acids of an amino acid sequence corresponding to the sequence of SEQ ID No: 5 in the above peptide are deleted, substituted, and/or added and which has the ability to improve tumor immunogenicity; and
   peptide (6): a peptide containing the amino acid sequence SQPLPNKIGF (SEQ ID No: 6), or a peptide in which one to several amino acids of an amino acid sequence corresponding to the sequence of SEQ ID No: 6 in the above peptide are deleted, substituted, and/or added and which has the ability to improve tumor immunogenicity.
<5> The pharmaceutical composition according to clause <4>, wherein the peptide is at least one of the peptides (1) and (2).
<6> A pharmaceutical composition containing, as an active ingredient, a splicing-controlling compound that causes a tumor to increase production of a peptide selected from the group consisting of the peptides (1) to (6) defined in clause <4> and combinations of two to six of these peptides.
<7> A pharmaceutical composition containing, as an active ingredient, a splicing-controlling compound that causes a tumor to increase production of at least one of the peptides (1) and (2) defined in clause <4>.
<8> The pharmaceutical composition according to any one of clauses <1> to <7>, wherein the splicing-controlling compound is not a splicing-controlling compound that modulates the splicing factor RBM39.
<9> The pharmaceutical composition according to any one of clauses <1> to <8>, wherein the splicing-controlling compound is at least one compound selected from the group consisting of compounds represented by the formulae (I), (I'), and (II) above and pharmaceutically acceptable salts thereof.
<10> The pharmaceutical composition according to any one of clauses <1> to <8>, wherein the splicing-controlling compound is at least one compound selected from the group consisting of compounds represented by the formulae (III) to (VII) above and pharmaceutically acceptable salts thereof.
<11> A pharmaceutical composition containing, as an active ingredient, at least one selected from the group consisting of the peptides (1) to (6) defined in clause <4> and combinations of two to six of these peptides, and pharmaceutically acceptable salts thereof.
<12> The pharmaceutical composition according to any one of clauses <6> to <10>, which is a pharmaceutical composition for improving tumor immunogenicity.
<13> The pharmaceutical composition according to any one of clauses <1> to <12>, which is administered in combination with an immune checkpoint inhibitor simultaneously, separately, or sequentially.
<14> The pharmaceutical composition according to clause <13>, wherein the immune checkpoint inhibitor is one or more agents against a molecule selected from the group consisting of (1) CTLA-4, (2) PD-1, (3) LAG-3, (4) BTLA, (5) KIR, (6) TIM-3, (7) PD-L1, (8) PD-L2, (9) B7-H3, (10) B7-H4, (11) HVEM, (12) GAL9, (13) CD 160, (14) VISTA, (15) BTNL2, (16) TIGIT, (17) PVR, (18) BTN1A1, (19) BTN2A2, (20) BTN3A2, and (21) CSF-1R.
< 15> A method for improving tumor immunogenicity, including bringing a splicing-controlling compound that modulates activity of serine/arginine-rich splicing factors (SRSFs) into contact with a tumor.
<16> A method for improving tumor immunogenicity, including bringing the pharmaceutical composition according to any one of clauses <1> to <14> into contact with a tumor.
<17> A method for enhancing immune checkpoint therapy, including administering simultaneously, separately, or sequentially an immune checkpoint inhibitor and the pharmaceutical composition according to any one of clauses <1> to <14> in combination.
<18> A kit for use in cancer immunotherapy, including the pharmaceutical composition according to any one of clauses <1> to <14> and an immune checkpoint inhibitor.
19> Use of a combination of the pharmaceutical composition according to any one of clauses <1> to <14> and an immune checkpoint inhibitor, for cancer immunotherapy.
<20> Use of a splicing-controlling compound that modulates activity of serine/arginine-rich splicing factors (SRSFs), for production of a pharmaceutical composition for improving tumor immunogenicity.
<21> A method for producing a splice-neoantigen, including bringing a tumor and a splicing-controlling compound that modulates activity of serine/arginine-rich splicing factors (SRSFs) into contact with each other.
<22> A pharmaceutical composition for use in improving immunogenicity of a tumor, the composition containing, as an active ingredient, a splice-neoantigen produced by the tumor in contact with a splicing-controlling compound that modulates activity of serine/arginine-rich splicing factors (SRSFs).

### Examples

Hereinafter, the present disclosure will be described in greater detail by means of examples, but these examples are illustrative, and the present disclosure is not limited to these examples. Note that all of the references cited in the present disclosure are incorporated as part of the present disclosure.

Compound 1 below is one of splicing modulators (also called "small-molecule splice modifier") targeting serine/arginine-rich splicing factors (SRSFs). Compound 1 shows the potential as a therapeutic agent for a genetic disease associated with aberrant pre-mRNA splicing (Nat Commun. 2011;2:308., Proc Natl Acad Sci USA. 2015 Mar 3;112(9):2764-9., Sci Rep. 2017 May 30;7:46126., J Clin Invest. 2019 Feb 1;129(2):583-597., Cell Chem Biol. 2020 Dec 17;27(12):1472-1482.e6.). In particular, it was recently reported that Compound 1, a potent RNA splicing modulator, was able to improve, in vitro and in vivo, aberrant splicing in the IKBKAP gene with IVS20+6T>C mutation, which causes familial dysautonomia, by modulating the activity of SRSFs (Nat Commun. 2021 Jul 23; 12(1):4507.).

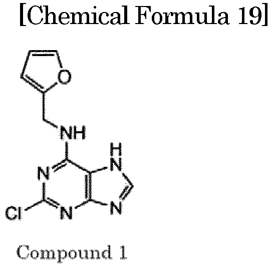

In the examples below, the following was performed.

First, to confirm whether Compound 1, a splicing modulator targeting SRSFs, suppresses tumor growth by enhancing antitumor immunity, cancer-bearing animal experiments using immunodeficient mice were conducted. It was confirmed that administration of Compound 1 suppressed tumor growth in a CD8⁺ T cell- and tumor MHC I-dependent manner and also enhanced PD-1 blockade efficacy.

Next, exploration of a neoantigen that can be induced using Compound 1 was attempted. To address this issue, RNA-seq was performed to extract non-annotated splice variants induced or enhanced by Compound 1 and create abnormal peptides translated from these novel variants. Immunogenic peptides were screened and identified from the extracted neoantigen candidates using an IFN-y ELISpot assay.

In subsequent in vivo vaccination studies, immunization with the chemically inducible splice-neoantigens elicited endogenous T cell responses for tumor lysis under co-culture conditions, and therefore it was revealed that sensitization of cancer cells with Compound 1 suppresses tumor growth.

Details are given below.

### <Compound 1 exhibits CD8⁺ T cell-dependent antitumor effects and potentiates the response to immune checkpoint therapy>

Conventional cytotoxic chemotherapy, which directly kills tumor cells, has been used to treat malignant tumors, but its use is often limited due to serious adverse effects. It was confirmed, in the following manner, that manipulating RNA splicing events using Compound 1, a small-molecule RNA splicing modulator, can enhance tumor immunogenicity and potentiate the response to immune checkpoint therapy that has no direct cytotoxic effects.

First, in vivo activity was assessed using syngeneic MC38 tumors intradermally transplanted into immunodeficient mice (FIG. 1-1A). Compound 1 resulted in in vivo tumor growth suppression and prolonged survival (FIGS. 1-1B and 1-1C) at concentrations that were not associated with cytotoxicity or cytostasis in vitro (FIGS. 1-1D and 1-1E).

It was found through analysis of extracted tumors that the frequency of tumor-infiltrating CD8⁺ T cells was increased to about 1.5-fold after Compound 1 treatment (FIGS. 1-1F and 1-1GU).

Based on these observations, whether the Compound 1 induced antitumor effects were due to enhanced cancer immunity was then examined. Endogenous CD8⁺ T cells were depleted using CD8-depleting antibody (αCD8), and the antitumor effects of Compound 1 administration were evaluated (FIG. 1-1H). An 80% or more depletion of CD8⁺ T cells was achieved in the spleen (FIGS. 1-2I and 1-2J) and tumor (FIGS. 1-2K and 1-2L), and it was found that Compound 1 administration was not effective for tumor suppression when CD8⁺ T cells were depleted (FIGS. 1-2M and 1-2N). This indicated that the antitumor activity of Compound 1 in the MC38 tumor-bearing model was attributed to enhanced CD8⁺ T cell-mediated immune responses.

As cancer immunogenicity has been associated with clinical responses to PD-1 blockade, whether Compound 1 treatment enhanced the antitumor effects of anti-PD-L1 antibody (αPD-L1) was determined.

It was found that, on day 23, Compound 1 alone suppressed tumor growth by 28.5% (isotype IgG + vehicle vs. isotype IgG + Compound 1, p = 1.1×10⁻³), whereas αPD-L1 suppressed tumor growth by 41.0% (isotype IgG + vehicle vs. αPD-L1 + vehicle, p < 1.0×10⁻⁴) (FIGS. 1-20 and 1-2P). Thus, a tumor suppression of about 57.8% is expected when Compound 1 and αPD-L1 function additively.

However, when Compound 1 and αPD-L1 were co-administered, a tumor suppression of 67.8% was observed (isotype IgG + vehicle vs. αPD-L1 + Compound 1, p ≤ 1.0×10⁻⁴), suggesting that they function additively or somewhat synergistically (FIGS. 1-2O and 1-2P).

Flow cytometry also revealed an increase in the number of tumor-infiltrating CD8⁺ T cells in a group co-treated with Compound 1 and αPD-L1, compared to a group treated with αPD-L1 alone. In addition, it was confirmed that collected tumor mass correlated well with measured tumor volumes in these experiments. However, there was no change in body weight or apparent adverse effects in the mice treated with isotype IgG, αPD-L1, or αCD8, with or without Compound 1 (FIG. 1-2Q).

These results indicated that Compound 1-induced chemical activation of SRSFs exerts antitumor effects in a CD8⁺ T cell-dependent manner and improves the efficacy of PD-1 blockade without direct cytotoxic or cytostatic effects on cancer cells.

### <Compound 1 does not induce direct activation of T cells or autoimmune reactions>

It seems likely that alterations in the production of splice isoforms during T cell activation affect the subsequent functions. After it was confirmed that Compound 1 exerts antitumor effects in a CD8⁺ T cell-dependent manner, whether Compound 1 induces antigen-independent activation of T cells was then examined using an ex vivo splenocyte culture assay.

First, splenocytes were isolated from normal mice, and the cells were treated with Compound 1 for 24 hours (FIG. 2- 1A). Then, the frequencies of the CD44⁺ effector T cell marker and CD69⁺ acute T cell activation marker in CD8⁺ or CD4⁺ T cells were measured using flow cytometry (FIGS. 2-1B and 2- 1C). As expected, treatment with concanavalin A (ConA) promoted both CD44⁺ and CD69⁺ marker expressions in CD8⁺ and CD4⁺ T cells, whereas treatment with Compound 1 had no such effects (FIGS. 2- 1C to 2-1E). Secretion of inflammatory cytokines, including interferon-y (IFN-γ) and tumor necrosis factor-a (TNF-α), was not observed in the Compound 1-administered group (FIGS. 2-1F and 2-1G).

These results excluded involvement of Compound 1 administration in antigen-independent activation of T cells.

Next, to determine whether the enhancement of cancer immunity by co-administration of Compound 1 and αPD-L1 raises concerns regarding autoimmune reactions, a high dose of Compound 1 was systemically administered to normal mice for 4 weeks (FIG. 2-2H).

Body weight changes were found to be comparable in all groups throughout the experimental period (FIG. 2-2I). With respect to acute reactions on day 7 after the initial administration, it was confirmed that Compound 1 administration with or without αPD-L1 had no effect on the spleen weight, leukocyte numbers, and expression of T cell activation markers (CD₄₄^{high}/CD8⁺, CD69+/CD8+, CD₄₄^{high}/CD₄⁺, CD69⁺/CD4⁺) in the spleen and peripheral blood (FIGS. 2-2J and 2-2K).

Furthermore, histological analysis of non-malignant tissues obtained on day 28 revealed that chronic exposure of normal mice to Compound 1 did not result in the accumulation of CD8⁺ T cells or appearance of inflammatory lesions, irrespective of co-treatment with αPD-L1 (FIGS. 2-3L and 2-3M). In addition, no alterations were observed in leukocyte numbers and biochemical parameters in peripheral blood (data not shown).

These results indicated that Compound 1 enhances cancer immunogenicity without adverse inflammatory or autoimmune reactions.

### <MHC class I in cancer cells was indispensable for antitumor effects of Compound 1>

MHC class I expressed in cancer cells is critical for neoantigen presentation, which is indispensable for CD8⁺ T cell-dependent immune surveillance and tumor killing. To investigate whether Compound 1 modulates the neoantigen presentation pathway, first, the effects of Compound 1 on IFN-y signaling and the expression of MHC class I components, including β2-microglobulin (B2M) and H2-K^{b} in MC38 cells, were examined because CD8⁺ T cell-secreted IFN-y is a major inducer of MHC class I components. Administration of IFN-y robustly induced the phosphorylation of STAT1 and STAT3 and increased the expression of the B2M protein, whereas administration of Compound 1 had no such effects. Also, a slight increase in H2-K^{b} expression (11.1% and 12.4% after administration of IFN-y at concentrations of 0 and 0.1 ng/mL for 72 hours) and a decrease in PD-L1 expression on the cell surface (3.8%, 3.2%, and 26.1% after administration of IFN-y at concentrations of 0, 0.1, and 1 ng/mL for 72 hours) were observed. In contrast, any changes in the expression of PD-L1 were not detected in host dendritic cells or macrophages. According to the RNA-seq data, mRNA expression and splicing patterns of H2-K^{b} and PD-L1 were not significantly altered in MC38 cells following treatment with Compound 1, and therefore, it was determined that these alterations were attributed to post-translational regulation. However, these alterations were likely not the functional mechanisms through which Compound 1 exerts its antitumor effects (FIGS. 1-2O and 1-2P), as enhanced antitumor effects of Compound 1 were observed when co-administered with αPD-L1.

Furthermore, whether neoantigen presentation in cancer cells is important for the antitumor effects of Compound 1 was investigated by establishing a CRISPR-Cas9-mediated knockout of B2m, an essential component of MHC class I, using MC38 cells. The resulting B2m-knockout MC38 clones (B2m KO #1 and #2) exhibited a complete loss of endogenous B2m without affecting cell proliferation (FIG. 3- 1A). It was further confirmed that surface H-2K^{b} on these cells disappeared (FIGS. 3- 1C and 3-1D) without affecting IFN-y signaling (FIG. 3- 1A) and the expression of PD-L1 on the cell surface (FIGS. 3-1E and 3-1F).

Then, these B2m KO MC38 clones were intradermally inoculated into immunodeficient mice, and the antitumor effects of Compound 1 were evaluated (FIG. 3-2G). Administration of Compound 1 consistently suppressed tumor growth in the negative control clone, whereas this effect was completely abolished in the B2m KO clones (FIG. 3-2H).

Moreover, the number of CD8⁺ T cells was lower in the B2m KO tumors, consistent with the essential role of MHC class I in promoting T cell proliferation, and was no longer inducible by treatment with Compound 1 in the B2m KO-derived tumors (FIGS. 3-2I and 3-2J). The antitumor effects of αPD-L1 were confirmed in the negative control clone, but not in the B2m KO clone (FIG. 3-2K).

These results clearly indicate that, in cancer cells, MHC class I plays a crucial role in determining the antitumor effects of Compound 1.

### <Compound 1 modulates RNA splicing and creates potential splice-neoantigens>

Aberrant selective splicing events in cancer cells may become a source of potential neoantigens. As it was confirmed that MHC class I in cancer cells is indispensable for the antitumor effects of Compound 1, Compound 1 was predicted to alter aberrant alternative splicing events and enhance the production of splice-neoantigens (FIG. 4- 1A). To validate this concept, RNA-seq analysis was performed to assess RNA splicing patterns affected by Compound 1 in MC38 cells and splice products yielding potential neoantigens capable of binding to MHC class I (FIG. 4- 1B). First, splicing events significantly altered by Compound 1 were extracted. Next, to identify neoepitopes translating potential splice-neoantigens, splicing events elicited by Compound 1 that created abnormal coding sequences were extracted, and subsequently, whether these events create abnormal peptides were checked by comparing them with a reference peptide database.

Among all the splicing events that were significantly affected by Compound 1, the events that were responsible for creating abnormal coding sequences with non-annotated epitopes not registered in both UniProt and Ensembl were focused on to identify splice-neoantigen candidates. With this approach, 169 Compound 1-induced non-annotated splice events for 130 genes were identified as neoantigen candidates, which were further subjected to prioritization based on the prediction scores for the binding affinity of each peptide to MHC class I alleles (H2-K^{b} and H2-D^{b}) with NetMHCpan4.1, the extents of splice rates, and mRNA expression (FIG. 4-2C).

Finally, 22 Compound 1-induced splice-neoantigen candidates (8 to 11 amino acids in length) were selected for subsequent evaluation (FIG. 4-2D and Table 1).

An example thereof is neurofibromin 1 (Nf1), in which treatment with Compound 1 induced the exonization of 108 bp, with the resulting splice product encoding the "RNRSHIFPL" candidate neoepitope, previously non-annotated and with a predicted affinity for H2-K^{b} at 238.15 nM (FIG. 4-2E). RNA-seq analysis also revealed that treatment with Compound 1 had no effect on the mRNA expression of genes encoding conventional neoantigens produced in MC38 cells (data not shown).

**[Table 1]**

| **Gene** | **Peptide** | **Event_Type** | **SEQ ID Nos** |
|---|---|---|---|
| Nf1 | RNRSHIFPL | Exon inclusion | 2 |
| Dop1a | DGPLFLKL | Alternative 5' splice site | 15 |
| Hacd1 | SSALFSYV | Alternative 5' splice site | 22 |
| Kifc1 | MALSNKAYV | Exon skipping | 1 |
| Igf2bp3 | QTQSNTEEI | Exon skipping | 12 |
| Qpctl | KQQELFVLL | Exon skipping | 5 |
| Acbd4 | SILGFTMV | Intron retention | 3 |
| Ric1 | VQFSAIDKL | Exon skipping | 21 |
| Pank4 | TAPSFLPL | Intron retention | 16 |
| Nup153 | SQPLPNKIGF | Exon skipping | 6 |
| Cep55 | STLHLSQAL | Exon skipping | 14 |
| Cep68 | AAGKKTQEL | Exon skipping | 17 |
| Rfx7 | SLLLLYLQL | Exon skipping | 4 |
| Gnpnat1 | FAKQEIELL | Exon skipping | 20 |
| Asah2 | IIYQKAKV | Exon skipping | 13 |
| Ap4e1 | TSLQNYKCTEC | Exon skipping | 7 |
| SIc38a2 | RVNFFAIL | Exon skipping | 11 |
| Nae1 | TELLSHTPL | Exon skipping | 19 |
| Septin10 | KSLDNKGHL | Exon skipping | 8 |
| Zmynd11 | QSFSQERL | Intron retention | 10 |
| Rabgap1 | SLYKICKM | Exon skipping | 18 |
| Nufip2 | SLSSNTSYGEI | Exon inclusion | 9 |

### <Validation of tumor-killing activity of Compound 1-induced immunogenic splice-neoantigens>

With the splice-neoantigen candidates obtained, whether Compound 1-induced splice-neoantigens bind to MHC class I and promote activation of CD8⁺ T cells was then experimentally validated.

The 22 splice-neoantigen candidates and a melanoma antigen peptide, tyrosinase-related protein 2 (TRP2)₁₈₀₋₁₈₈, serving as a positive control, were synthesized. Immunodeficient mice were vaccinated twice with the synthesized peptides in the presence of Poly(I:C) as an adjuvant. Then, splenocytes were prepared from the vaccinated mice and co-cultured with peptide-pulsed bone marrow-derived dendritic cells (BMDCs) for 24 hours, and the activation of CD8⁺ T cells was evaluated using an IFN-y ELISpot assay (FIG. 5-1A).

Of the 22 peptides examined, the INF-y ELISpot assay identified 6 peptides that were immunogenic and were derived from the altered splicing of Kifc1, Nf1, Acbd4, Rfx7, Qpctl, andNupl53 (FIGS. 5-1B and 5-1C).

In addition to the RNA-seq analysis, the induction of these splice-neoantigen-coding transcripts via treatment with Compound 1 was also validated using RTPCR analysis with neojunction primers in MC38 cells (FIGS. 5-2D and 5-2E). The immunogenicity of the identified splice-neoantigens was confirmed using an IFN-y ELISpot assay.

Then, whether the Compound 1-induced splice-neoantigens are shared across cancer type was examined using murine lung carcinoma LLC, pancreatic ductal adenocarcinoma Pan02, leukemia WEHI3, colorectal adenocarcinoma CT26, lymphoma EG7, and skin melanoma B 16 cells. Interestingly, it was found that these splice alterations induced by Compound 1 were shared in most of the examined cells (FIG. 5-2F), suggesting that Compound 1 is applicable to a broad spectrum of cancers.

In addition, the RNA-seq data corresponding to colon, lung, and liver tissues from Compound 1-administered mice was also examined to analyze the extent of splicing alterations. Compound 1-induced splicing events were found to be 50-60% fewer in normal tissues than in MC38 cells. Considering that immune responses were not triggered by Compound 1 in these tissues, these altered splicing events in normal tissues were likely irrelevant to immunogenicity (FIGS. 2-2I to 2-3M). In fact, it was found that none of the validated splice-neoantigen forms were significantly induced in normal tissues (data not shown).

The genes from which the six immunogenic peptides were derived and the amino acid sequences of these peptides are as follows.
Kifc1 gene: MALSNKAYV (SEQ ID No: 1)
Nf1 gene: RNRSHIFPL (SEQ ID No: 2)
Acbd4 gene: SILGFTMV (SEQ ID No: 3)
Rfx7 gene: SLLLLYLQL (SEQ ID No: 4)
Qpctl gene: KQQELFVLL (SEQ ID No: 5)
Nup 153 gene: SQPLPNKIGF (SEQ ID No: 6)

### <Hexavalent vaccination of inducible splice-neoepitopes promotes Compound 1-induced cancer immune responses>

As it was confirmed that Compound 1-inducible splice-neoantigens were immunogenic, their contribution to anticancer immune responses was then assessed. For this purpose, a co-culture assay was used to ensure tumor killing (FIG. 6-1A).

Splenocytes were prepared from mice vaccinated with either hexavalent splice-neoantigens or an irrelevant Tpr2₁₈₀₋₁₈₈ antigen, and then further stimulated with BMDCs pulsed with the same antigen(s) in the presence of IL-2. Tumor-killing activity was quantified based on the frequency of Annexin V⁺/CFSE⁺ 24 hours after co-culture with antigen(s)-elicited effector cells and CFSE-prelabeled target cells. Although the effector cells from the Tpr2-immunized mice did not exhibit any response, regardless of peptide loading, the hexavalent splice-neoantigen-stimulated effector cells selectively killed MC38 cells presenting six splice-neoepitopes (FIGS. 6-1B and 6-1C). As a result, this tumor-killing activity was absent for the B2m KO MC38 cells (FIG. 6-1D). This indicates that this effect was MHC class I-dependent.

In addition, contributions of the individual epitopes were also examined through co-culture assays for tumor killing, and it was observed that most of the epitopes (Kifc1, Nf1, Acbd4, Rfx7, and Nup 153) individually and significantly triggered the tumor-killing responses (FIG. 7A). In addition, when the tumor-killing responses were investigated in the context of cancer cells induced to produce endogenous splice-neoantigens through treatment with Compound 1, it was found that effector cells from hexavalent vaccine-immunized mice exhibited cytotoxic activity against all three cancer cells examined, namely, MC38 (FIG. 6-2E), B 16 (FIG. 6-2F), and LLC (FIG. 6-2G), all of which expressed splice-neoantigens in response to Compound 1 treatment (FIG. 5-2).

In contrast, these effector cells did not show cell lytic activity toward noncancerous mouse embryonic fibroblasts (MEFs) from B6 mice (FIG. 6-2H). Expression of splice-neoantigens was very low compared with MC38, suggesting that MEFs avoided lysis by CD8⁺ T cells owing to a lack of sufficient induction of splice-neoantigens.

Although inducible splicing-derived peptides in cancer cells were previously proposed, their importance in tumor growth in vivo remained uncharacterized. Thus, next, whether Compound 1-inducible splice-neoepitope-reactive T cells can suppress tumor growth was investigated (FIG. 6-2I).

The hexavalent vaccine its own resulted in suppressed tumor growth (Poly(I:C) + vehicle vs. Poly(I:C)/hexavalent peptide + vehicle, 24.7% suppression on day 23) (FIG. 6-2J). This is likely owing to the immunogenicity of basally expressed splice-neoantigens in cancer cells (FIGS. 5-2D and 5-2E), and this effect was further enhanced by co-administration with Compound 1 (Poly(I:C) + vehicle vs. Poly(I:C)/hexavalent peptide + Compound 1, 57.0% suppression on day 23) (FIG. 6-2J). Also, the effect of each splice-neoantigen by individual vaccination with Compound 1 was evaluated, and it was found that there was a 6% to 23% suppression in tumor growth compared with that in non-vaccinated controls (FIG. 7B). The tumor suppressive effects of individual vaccination were weaker than those of hexavalent vaccinations, which resulted in a suppression rate of 30.7% (FIG. 6-2J), indicating that the anticancer immune response was enhanced by simultaneous vaccination with the six identified neoepitopes.

### <RNA-Seq analysis using Compound 2>

Compound 2 shown below is one of splicing modulators targeting serine/arginine-rich splicing factors (SRSFs). Compound 2 has the function of modulating a Cdc2-like kinase (CLK) and affects SRSFs through the modulation of CLK (Cell chem biol., 2020, vol.27, P1472-1482).

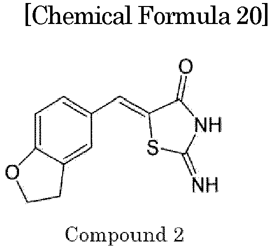

The effects of Compound 2 on RNA splicing events (splicing alterations) producing the aforementioned six splice-neoantigens in various murine cancer cell lines were investigated through RNA-Seq analysis in the same manner as in FIG. 5-2F, except that Compound 1 was replaced with Compound 2 (FIG. 8). FIG. 8 shows that Compound 2 increases the production of splice-neoantigens in the various murine cancer cell lines. In particular, splicing alterations of the Kifc1 gene that result in the production of peptides containing MALSNKAYV (SEQ ID No: 1) were markedly observed in the cancer cell line MC38 and the skin melanoma cell line B16, and splicing alterations of the Rfx7 gene that result in the production of peptides containing SLLLLYLQL (SEQ ID No: 4) were markedly observed in all the cancer cell lines.

### <Experimental Methods>

### ELISpot assay

Eight-week-old female C57BL/6N mice were subcutaneously injected with 100 µg of synthesized peptides (Genscript Japan) and 50 µg of poly(I:C) formulated in PBS (5 peptides/mouse) on days 0 and 7. H2-K^{b} TRP-2₁₈₀₋₁₈₈ peptide (MBL International, Woburn, MA) was used as a positive control. Mice were sacrificed 12 days after the initial injection, and splenocytes were isolated. As stimulators, bone marrow-derived dendritic cells (BMDCs) were generated as described previously, with some modifications. Briefly, bone marrow cells were isolated from femurs of the 8-week-old female C57BL/6N mice through centrifugation on day 2. Cells were passed through a 70-µm cell strainer and seeded in 10-cm petri dishes with RPMI1640 containing 10% FBS, 100 U/mL P/S, 2 mM L-glutamine, 50 pM 2-ME, and 20 ng/mL Gm-csf (Peprotech, Rocky Hill, NJ). Culture was maintained by replacing half of the medium every 2 or 3 days. On day 12, most of the nonadherent cells had acquired typical dendritic morphology, and these cells were pulsed with peptides (2 µg/ml) for 2 hours at 37°C. ELISpot assay was performed using a Mouse IFN-γ ELISpot BASIC kit (Mabtech AB, Nacka Strand, Sweden) as per the manufacturer's instructions. Freshly isolated splenocytes (5 × 10⁵ cells) were co-incubated with peptide-pulsed BMDCs (4 × 10⁴ cells) overnight at 37°C under 5% CO₂ conditions in an ELISpot PVDF white plate coated with anti-IFN-γ antibody (clone AN 18). Then, IFN-γ secretion was detected using a capture antibody (clone R4-6A2) and a BCIP/NBT-plus substrate. After leaving the plate to dry, the spot number was counted using a dissection microscope. Spot images were acquired using an ImmunoSpot S6 Ultimate Analyzer (Cellular Technology Limited, Cleveland, OH).

### Co-culture assay for the measurement of tumor-killing activity

Splenocytes were prepared from peptide(s)-vaccinated mice inoculated with Poly(I:C) on day 14, as described in the section of "ELISpot assay". To further elicit antigen-specific CD8⁺ T cells, freshly isolated splenocytes were co-cultured with peptide(s)-pulsed bone marrow-derived dendritic cells (BMDCs) in the presence of 10 ng/mL IL-2 (Peprotech) for another 7 days. Culture was maintained by replacing half of the medium every 2 or 3 days. Splenocytes stimulated with BMDCs, as effector cells, were then co-cultured with target cells at an effector target ratio of 5:1 for 24 hours to examine their lytic activity. For the preparation of target cells, in the previous day of co-culture with effector cells, cells were labeled with 2 M carboxyfluorescein diacetate succinimidyl ester (CFSE; Dojindo) for 10 minutes, and incubated with FBS for additional 10 minutes to stop the labeling reaction. After washing with medium twice, cells were cultured overnight in the presence of 10 ng/mL IFN-y. The next day, target cells were pulsed with 10 ng/mL peptide(s) for 2 hours prior to co-culture or treated with Compound 1 in the presence of effector cells. After 24 hours of co-culture, cells were stained with annexin V (Biolegend) in an annexin V binding buffer for 15 minutes and subjected to flow cytometry analysis. Effector cell-mediated killing activity was calculated from the frequencies of annexin V⁺/CFSE⁺.

### RT-PCR

Total RNA was extracted from cells using a RNeasy Mini Kit (Qiagen), according to the manufacturer's protocol, and subjected to reverse transcription using a High Capacity cDNAReverse Transcription Kit (Applied Biosystems, Foster City, CA). Genes were amplified with KOD One DNA polymerase (ToYoBo) and target-specific primer sets. RT-PCR products were separated using electrophoresis. Ethidium bromide-stained images were captured using a ChemiDoc MP Imaging System and the data were analyzed using Imaged software. The following primers were used: 5'-TTC CTG TGA GAAAGA GGT GGAG-3' (SEQ ID No: 23) and 5'-CAC AAA CAT AAG CCT TAT TGC TCA G-3' (SEQ ID No: 24) for Kife1; 5'-CAC TTT AGT GAA GAG ACC AAG CAA G-3' (SEQ ID No: 25) and 5'-TCA TAC ATG CTT GTT GTG GCA GAG-3' (SEQ ID No: 26) for Nfl; 5'-CAT GGG TAC CGA GAA GGAAGA G-3' (SEQ ID No: 27) and 5'-TCC AAT GGG GTC CCA GAA C-3' (SEQ ID No: 28) for Acbd4; 5'-AAA CTG GAG ATG GGG ACT TA-3' (SEQ ID No: 29) and 5'-TGA GGA TTC TCC TGG CAA TG-3' (SEQ ID No: 30) for Rfx7; 5'-CCAAGT GAG AAA GTT CCT GGA G-3' (SEQ ID No: 31) and 5'-GAG GAC AAA GAG CTG CTG CTG-3' (SEQ ID No: 32) for Qpctl; 5'-CCA GAG AAT GAG GAA GTG GAA G-3' (SEQ ID No: 33) and 5'-GTA AAT CCAATC TTG TTT GG-3' (SEQ ID No: 34) for Nup 153; and 5'-TCT TTG CAG CTC CTT CGT TG-3' (SEQ ID No: 35) and 5'-GGC CTC GTC ACC CAC ATA G-3' (SEQ ID No: 36) for Actb. Actb was used as a loading control.

### RNA-seq and identification of splice-neoantigen candidate

RNA-Seq libraries were prepared using a TruSeq Stranded mRNALibrary Kit (Illumina, San Diego, CA) and used for paired-end sequencing on an Illumina NovaSeq 6000 platform. RNA-seq data were mapped to the GRCm38 (mm10) genome sequence using HISAT2 program version 2.2.0 with the "--rna-strandness RF" option. Splice-junction information available from Ensembl gene annotation version 100 was used in this process. Mapped results were discarded when either of the paired reads was not mapped to the genome, or the relationships of mapping positions were abnormal.

For splicing analysis, rMATS program version 4.1.0 was used. For the reference transcript models used in the rMATS analysis, a set of new transcripts based on RNA-seq data was constructed using Cufflinks program version 2.2.1. Because the rMATS program skips soft-clipped reads and reads having indels in their alignment, bam files were modified to replace soft clipping and indel information as not changing the splice position information. After rMATS analysis, altered splicing events were extracted according to the following criteria: FDR < 0.01, dPSI ≥ 5.0, and average number of spliced reads ≥ 15 in either of the samples.

Spliced regions were compared with Ensembl gene annotations for describing the amino acid sequence encoded in these regions. Amino acid sequences not observed in the original peptide database were searched and stored with seven amino acids margins from known sequences.

For predicting affinities of splicing-derived peptides to MHC class I (H2-K^{b} and H2-D^{b}), NetMHCpan version 4.1 with the option "-1, 8, 9, 10, 11" was performed. For DEG analysis, transcripts per million (TPM) values and raw reads counts were calculated with RSEM program version 1.2.31. Then, DESeq2 program version 1.8.2 was used to find DEGs fulfilling the following criteria: in either of the samples, adjusted-p < 0.05, fold change ≥ 1.3 or ≤ 1/1.3, average TPM ≥ 1, and average raw-read counts ≥ 32, in samples.

## Claims

1. A pharmaceutical composition for use in improving tumor immunogenicity, comprising, as an active ingredient, a splicing-controlling compound that modulates activity of serine/arginine-rich splicing factors (SRSFs).

2. The pharmaceutical composition according to claim 1, wherein the compound is a compound capable of modulating a Cdc2-like kinase (CLK).

3. The pharmaceutical composition according to claim 1 or 2, wherein the compound is a compound that enhances production of a peptide that improves tumor immunogenicity in a tumor.

4. The pharmaceutical composition according to any one of claims 1 to 3, wherein the peptide is selected from the group consisting of the peptides (1) to (6) below and combinations of two to six of these peptides:
peptide (1): a peptide comprising the amino acid sequence MALSNKAYV (SEQ ID No: 1), or a peptide in which one to several amino acids of an amino acid sequence corresponding to the sequence of SEQ ID No: 1 in the above peptide are deleted, substituted, and/or added and which has the ability to improve tumor immunogenicity;
peptide (2): a peptide comprising the amino acid sequence RNRSHIFPL (SEQ ID No: 2), or a peptide in which one to several amino acids of an amino acid sequence corresponding to the sequence of SEQ ID No: 2 in the above peptide are deleted, substituted, and/or added and which has the ability to improve tumor immunogenicity;
peptide (3): a peptide comprising the amino acid sequence SILGFTMV (SEQ ID No: 3), or a peptide in which one to several amino acids of an amino acid sequence corresponding to the sequence of SEQ ID No: 3 in the above peptide are deleted, substituted, and/or added and which has the ability to improve tumor immunogenicity;
peptide (4): a peptide comprising the amino acid sequence SLLLLYLQL (SEQ ID No: 4), or a peptide in which one to several amino acids of an amino acid sequence corresponding to the sequence of SEQ ID No: 4 in the above peptide are deleted, substituted, and/or added and which has the ability to improve tumor immunogenicity;
peptide (5): a peptide comprising the amino acid sequence KQQELFVLL (SEQ ID No: 5), or a peptide in which one to several amino acids of an amino acid sequence corresponding to the sequence of SEQ ID No: 5 in the above peptide are deleted, substituted, and/or added and which has the ability to improve tumor immunogenicity; and
peptide (6): a peptide comprising the amino acid sequence SQPLPNKIGF (SEQ ID No: 6), or a peptide in which one to several amino acids of an amino acid sequence corresponding to the sequence of SEQ ID No: 6 in the above peptide are deleted, substituted, and/or added and which has the ability to improve tumor immunogenicity.

5. The pharmaceutical composition according to claim 4, wherein the peptide is at least one of the peptides (1) and (2).

6. A pharmaceutical composition comprising, as an active ingredient, a splicing-controlling compound that enhances production of a peptide selected from the group consisting of the peptides (1) to (6) defined in claim 4 and combinations of two to six of these peptides in a tumor.

7. A pharmaceutical composition comprising, as an active ingredient, a splicing-controlling compound that enhances production of at least one of the peptides (1) and (2) defined in claim 4 in a tumor.

8. The pharmaceutical composition according to any one of claims 1 to 7, wherein the splicing-controlling compound is not a splicing-controlling compound that modulates a splicing factor RBM39.

9. The pharmaceutical composition according to any one of claims 1 to 8, wherein the splicing-controlling compound is at least one compound selected from the group consisting of compounds represented by the formulae (I) to (II) below and pharmaceutically acceptable salts thereof 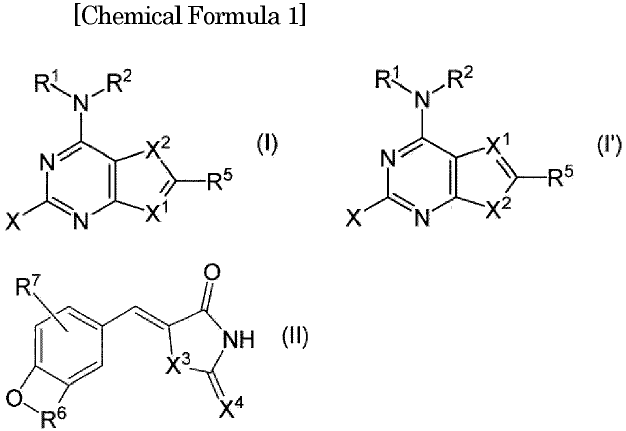 where, in the formulae (I) and (I'),
R¹ and R² each independently represent a hydrogen atom, a substituted or unsubstituted C₁-C₆ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, R¹ and R² are bonded to each other to form a ring together with N, and the ring is a substituted or unsubstituted monocyclic heterocyclic ring or a substituted or unsubstituted bicyclic heterocyclic ring,
R⁵ represents a hydrogen atom, a halogen atom, a substituted or unsubstituted C₁-C₆ alkoxy group, or a dialkylamino group;
X¹ represents N or -CH-;
X² represents -N(R³)-, S, or O;
R³ represents a hydrogen atom, a C₁-C₆ alkyl group, a benzyl or heteroarylmethyl group, a substituted or unsubstituted aryl group, a substituted or unsubstituted heteroaryl group, or CH₂OC(O)R⁴-;
R⁴ represents a C₁-C₆ alkyl group, a benzyl or heteroarylmethyl group, a substituted or unsubstituted aryl group, or a substituted or unsubstituted heteroaryl group; and
X represents a hydrogen atom, a halogen atom, an amino group, an amino group substituted with R¹ and R², an azido group, a cyano group, a nitro group, a hydroxy group, a C₁-C₆ alkyloxy group, a substituted or unsubstituted aryloxy group, a substituted or unsubstituted heteroaryloxy group, a mercapto group, a C₁-C₆ alkylthio group, a substituted or unsubstituted arylthio group, a substituted or unsubstituted heteroarylthio group, a benzyl or heteroarylmethyl group, a substituted or unsubstituted aryl group, or a substituted or unsubstituted heteroaryl group, and
in the formula (II),
X³ and X⁴ each independently represent S or NH;
R⁶ represents 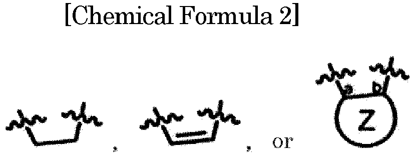
where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being hydrogen, a halogen atom, or a C₁-C₆ alkyl group; and
R⁷ represents a hydrogen atom, a halogen atom, or a C₁-C₆ alkyl group.

10. The pharmaceutical composition according to any one of claims 1 to 8, wherein the splicing-controlling compound is at least one compound selected from the group consisting of compounds represented by the formulae (III) to (VII) below and pharmaceutically acceptable salts thereof 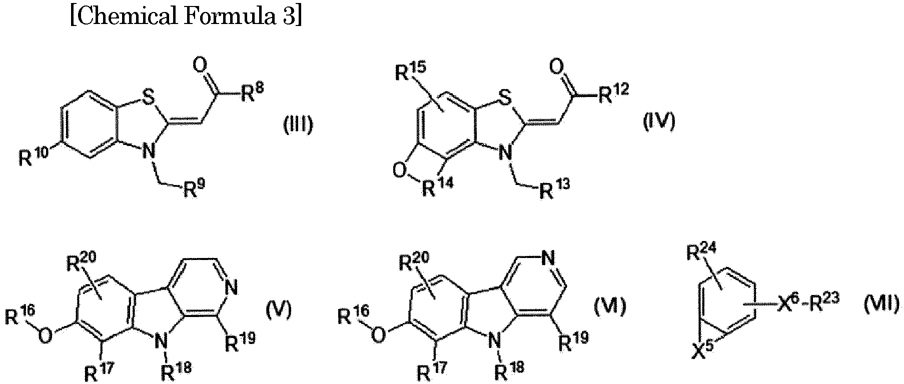 where, in the formula (III),
R⁸ and R⁹ each independently represent a hydrogen atom, a halogen-substituted or unsubstituted C₁-C₁₀ alkyl group, or a C₂-C₆ alkenyl group;
R¹⁰ represents a hydrogen atom, a halogen atom, or a halogen-substituted or unsubstituted C₁-C₁₀ alkyl group, -OR¹¹, -NHR¹¹, or -N(R¹¹)₂; and
R¹¹ represents a hydrogen atom or a C₁-C₁₀ alkyl group,
in the formula (IV),
R¹² and R¹³ each independently represent a hydrogen atom or a C₁-C₆ alkyl group;
R¹⁴ represents 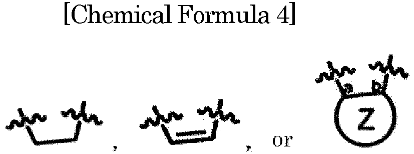
where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being hydrogen, a halogen atom, or a C₁-C₆ alkyl group; and
R¹⁵ represents a hydrogen atom, a halogen atom, or a C₁-C₆ alkyl,
in the formulae (V) and (VI),
R¹⁶ and R¹⁸ each independently represent a hydrogen atom, a C₁-C₆ alkyl group, a benzyl or heteroarylmethyl group, a substituted or unsubstituted aryl group, or a substituted or unsubstituted heteroaryl group;
R¹⁷ represents -R²¹, -C≡C-R²¹, -CH=CH-R²¹, or -O-(CH₂)n-R²¹, where n represents 1 to 6, and R²¹ represents a hydrogen atom, a hydroxy group, a C₁-C₈ alkyl group, -Si(R²²)₃, or a substituted or unsubstituted phenyl group, a monocyclic heteroaromatic ring group, or a cycloaliphatic group;
alternatively, R¹⁶ and R¹⁷ are bonded to each other to form a ring, and -R¹⁶-R¹⁷-represents -(CH₂)m-CH₂-, -CH=CH-, -(CH₂)m-O-, or any of these substituted with a halogen atom, where m represents 1 to 6, R²² represents a hydrogen atom, a C₁-C₆ alkyl group, a trihalomethyl group, or a hydroxy group, and the three atoms or groups R²² in -Si(R²²)₃ may be different from one another; and
R¹⁹ and R²⁰ represent hydrogen atoms or C₁-C₆ alkyl groups, and
in the formula (VII),
X⁵ represents 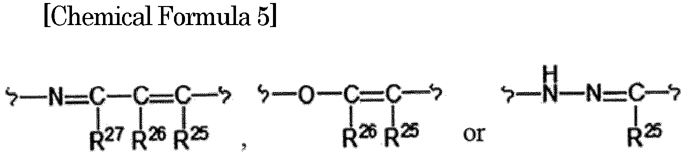
where R²⁵, R²⁶, and R²⁷ each independently represent hydrogen, a halogen atom, a carboxyl group, an amino group, a hydroxy group, a C₁-C₄ alkyl group, or a C₁-C₄ alkyl group substituted with a halogen atom;
X⁶ represents -(bond) or -NH-;
R²³ represents 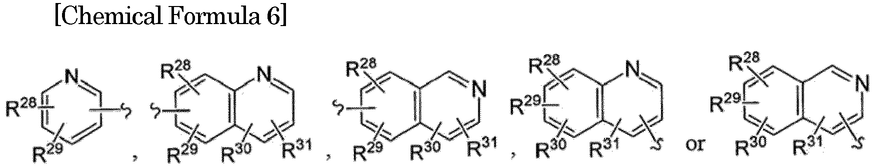
where R²⁸, R²⁹, R³⁰, and R³¹ each independently represent hydrogen, a halogen atom, a carboxyl group, an amino group, a hydroxy group, a C₁-C₄ alkyl group, or a C₁-C₄ alkyl group substituted with a halogen atom; and
R²⁴ represents a hydrogen atom, a halogen atom, a carboxyl group, an amino group, a hydroxy group, or a halogen-substituted or unsubstituted C₁-C₄ alkyl group.

11. A pharmaceutical composition comprising, as an active ingredient, at least one selected from the group consisting of the peptides (1) to (6) defined in claim 4 and combinations of two to six of these peptides, and pharmaceutically acceptable salts thereof.

12. The pharmaceutical composition according to any one of claims 6 to 11, which is a pharmaceutical composition for use in improving tumor immunogenicity.

13. The pharmaceutical composition according to any one of claims 1 to 12, which is administered in combination with an immune checkpoint inhibitor simultaneously, separately, or sequentially

14. The pharmaceutical composition according to claim 13, wherein the immune checkpoint inhibitor is one or more agents against a molecule selected from the group consisting of (1) CTLA-4, (2) PD-1, (3) LAG-3, (4) BTLA, (5) KIR, (6) TIM-3, (7) PD-L1, (8) PD-L2, (9) B7-H3, (10) B7-H4, (11) HVEM, (12) GAL9, (13) CD 160, (14) VISTA, (15) BTNL2, (16) TIGIT, (17) PVR, (18) BTN1A1, (19) BTN2A2, (20) BTN3A2, and (21) CSF-1R.

15. A method for improving tumor immunogenicity, comprising bringing a splicing-controlling compound that modulates activity of serine/arginine-rich splicing factors (SRSFs) into contact with a tumor.

16. A method for improving tumor immunogenicity, comprising bringing the pharmaceutical composition according to any one of claims 1 to 14 into contact with a tumor.

17. A method for enhancing immune checkpoint therapy, comprising administering simultaneously, separately, or sequentially an immune checkpoint inhibitor and the pharmaceutical composition according to any one of claims 1 to 14 in combination.

18. A kit for use in cancer immunotherapy, comprising the pharmaceutical composition according to any one of claims 1 to 14 and an immune checkpoint inhibitor.

19. Use of a combination of the pharmaceutical composition according to any one of claims 1 to 14 and an immune checkpoint inhibitor, for cancer immunotherapy.

20. Use of a splicing-controlling compound that modulates activity of serine/arginine-rich splicing factors (SRSFs), for production of a pharmaceutical composition for use in improving tumor immunogenicity.

21. A method for producing a splice-neoantigen, comprising bringing a tumor and a splicing-controlling compound that modulates activity of serine/arginine-rich splicing factors (SRSFs) into contact with each other.

22. A pharmaceutical composition for use in improving immunogenicity of a tumor, comprising, as an active ingredient, a splice-neoantigen produced by the tumor in contact with a splicing-controlling compound that modulates activity of serine/arginine-rich splicing factors (SRSFs).
